# EUROPEAN PATENT APPLICATION

(11) **EP 4 748 400 A1**
(43) Date of publication of application: **27.05.2026**
(21) Application number: 24849529.3
(22) Date of filing: 26.07.2024
(51) Int. Cl.: A61K 47/64, A61K 39/385, A61P 37/00, A61P 35/00, A61K 39/00

(54) **CONJUGATE FOR ANTIGEN DELIVERY AND USE THEREOF**

(30) Priority: 28.07.2023 KR 20230099234; 04.08.2023 KR 20230102465
(71) Applicant: LG Chem, Ltd., Seoul 07336 (KR)
(72) Inventor: KIM, Dae Hee, Daejeon 34122 (KR); MIN, Kyeongsik, Daejeon 34122 (KR); KWON, Joon Cheol, Daejeon 34122 (KR); KIM, Seunghae, Daejeon 34122 (KR); KIM, Da Eun, Daejeon 34122 (KR); KIM, Hyeongsu, Daejeon 34122 (KR)
(74) Representative: Goddar, Heinz J.
(86) International application number: PCT/KR2024/010983
(87) International publication number: WO 2025/028960

(57) **Abstract**

The present invention relates to: a conjugate comprising mRNA of an antigen protein and mRNA encoding a carrier protein linked to the 5' end and the 3' end of the mRNA of the antigen protein; and an immunization composition and/or vaccine composition comprising same. The present invention has the effect of stably increasing the expression of the antigen protein.

## Description

### [TECHNICAL FIELD]

### Cross-reference to related applications

The present application claims the benefit of priority based on Korean patent application No. 10-2023-0099234 filed on July 28, 2023 and Korean patent application No. 10-2023-0102465 filed on August 4, 2023, and the entire contents disclosed in the documents of the corresponding Korean patent applications are incorporated as part of this specification.

The present description relates to a conjugate comprising mRNA of an antigen protein, and mRNA encoding a carrier protein linked to the 5' end and/or 3' end of the mRNA of the antigen protein, and a composition for immunization and/or a vaccine composition comprising thereof.

### [BACKGROUND ART]

An anticancer treatment vaccine is a method of treating cancer, by strengthening or inducing an antigen-specific immune response. Conventional anticancer treatment vaccines do not reflect the antigen processing process, so the expression level is not constant or high depending on the antigen sequence, and therefore, a specific immune response against antigen sequences different from each other is not high or it is difficult to expect constant therapeutic efficacy.

In order to express various antigen sequences without problems by solving the above problems, it was tried to induce a high immune response by increasing the expression level, reduce the deviation of the amount of antigen proteins expressed by various antigens, and appropriately deliver CD4/CD8 antigens to a processing/presentation pathway, respectively, when an antigen is delivered in an mRNA form into a cell, by linking mRNA sequences encoding different proteins (carrier proteins) at both the 5' and 3' ends of the antigen mRNA sequence.

### [DISCLOSURE]

### [TECHNICAL PROBLEM]

The present description provides a conjugate comprising mRNA of an antigen protein and mRNA encoding a first carrier protein linked to the 5' end and/or 3' end of the mRNA of the antigen protein, and increases the expression level of the antigen protein.

Another embodiment provides a composition for immunization and/or a vaccine composition, comprising the conjugate.

Other embodiment provides a method for immunization, comprising administering the conjugate and a composition for immunization and/or vaccine composition comprising thereof into a subject.

### [TECHNICAL SOLUTION]

One embodiment provides a conjugate, comprising mRNA of an antigen protein, and mRNA encoding a carrier protein linked to the 5' end, 3' end, or both of the mRNA of the antigen protein, and
specifically, provides a conjugate, comprising mRNA of an antigen protein;
mRNA encoding a first carrier protein linked to the 5' end of the mRNA of the antigen protein; and
mRNA encoding a second carrier protein linked to the 3' end of the mRNA of the antigen protein.

The first carrier protein and the second carrier protein may be same or different, but not limited thereto.

The first carrier protein and the second carrier protein may be at least one selected from Ub (ubiquitin), UbR (ubiquitin A77R mutant), Lamp, mODC (mouse ornithine decarboxylase), cmyc, SORT1 (sortilin 1), TRX (Thioredoxin) (for example, TRXwt and/or TRXmt), PSBD (peripheral subunit-binding domain), TRX-UbR (for example, TRXwt-UbR), TRX-Lamp (for example, TRXwt-Lamp), TRX-mODC (for example, TRXwt-mODC), TRX-cmyc (for example, TRXwt-cmyc), TRX-SORT1 (for example, TRXwt-SORT1), SORT1 w/TM and Lamp w/TM, respectively, but not limited thereto.

The antigen protein may comprise a T cell epitope, but not limited thereto.

The conjugate may have at least one characteristic selected from the group consisting of the following (i) and (ii) by the carrier protein, but not limited thereto:
(i) delivery of CD4/CD8 through a processing and presentation pathway, respectively; and
(ii) increase in expression of the antigen protein.

Another embodiment provides a composition for immunization and/or a vaccine composition, comprising the conjugate.

Other embodiment provides a method for immunization against cancer, comprising administering the conjugate and/or a composition for immunization and/or a vaccine composition comprising the same into a subject in need of immunization against cancer.

Other embodiment provides a composition for preventing, improving and/or treating cancer, infectious disease, autoimmune diseases and/or allergic disease.

Other embodiment provides a use for using the conjugate and/or composition in immunization against cancer or a use for using in preparation of the composition (for example, composition for immunization and/or vaccine composition) used for immunization against cancer.

Other embodiment provides a use for using the conjugate and/or composition in prevention, improvement and/or treatment of cancer, infectious disease, autoimmune disease and/or allergic disease, or a use for using in preparation of the composition used for prevention, improvement, and/or treatment of cancer, infectious disease, autoimmune disease and/or allergic disease (for example, composition for prevention, improvement, and/or treatment of cancer, infectious disease, autoimmune disease, and/or allergic disease).

Hereinafter, the present invention will be described in more detail below.

### Conjugate

One embodiment of the present description provides a conjugate, comprising mRNA of an antigen protein;
mRNA encoding a first carrier protein linked to the 5' end of the mRNA of the antigen protein; and
mRNA encoding a second carrier protein linked to the 3' end of the mRNA of the antigen protein.

In the present description, "antigen" refers to any molecule that induces an immune response in an object. The antigen refers to any molecule which can be recognized by a T cell receptor and/or a B cell receptor, and can stimulate an immune response, in particular, a T cell response and/or a B cell response in an object.

In the present description, "epitope" refers to a region of an antigen that interacts with a T-cell receptor and/or B-cell receptor.

In the present description, "carrier protein" refers to a protein which is linked to an antigen protein, and improves recombinant expression of an antigen protein (for example, high expression rate, consistent expression rate, etc.), improves purification, enhances physical properties, stabilizes, increases immunogenicity, and/or improves delivery to immune response cells. In the present description, the carrier protein may be in a form of full-length protein and/or in a form comprising some sequences of the amino acid sequence of the full-length protein, but not limited thereto. In the present description, the carrier protein may be linked to the N-terminus and C-terminus of the antigen protein, respectively, and each corresponds to the first carrier protein and the second carrier protein.

In the present description, "linked" refers to that elements are directly or indirectly linked together. Each element may be covalently or non-covalently linked. In addition, "linked" may mean that chemical or physical bonds of each of the elements are maintained, after contact and immunization with cells, for example, antigen-presenting cells or immunocytes. For example, when the antigen-presenting cells and immunocytes come in contact, they may be associated so that they do not freely disperse from one another. For example, two components may be covalently linked to each other, so that these two components cannot be dispersed or diffused separately.

A linked may be present between the mRNA of the antigen protein of the conjugate and mRNA encoding the first carrier protein and/or mRNA encoding the second carrier protein, but it is not limited thereto.

In the present description, "linker" refers to a molecule or atom group which links or couples or binds two or more components together. In the present description, each component of the antigen protein, for example, the antigen protein and carrier protein, may be linked or bound together by any appropriate means, respectively.

The linker may be (GS)n, (G₂S)n, (G₃S)n, (G₄S)n, Gn, LE, SSGG or GGGGSGGGGG (herein, G is Gly, S is Ser, L is Leu, E is Glu, n is an integer of at least 1), but not limited thereto.

### Carrier protein

In the conjugate, comprising mRNA of an antigen protein;
mRNA encoding a first carrier protein linked to the 5' end of the mRNA of the antigen protein; and
mRNA encoding a second carrier protein linked to the 3' end of the mRNA of the antigen protein, provided in the present description,
the first carrier protein and the second carrier protein may be same or different, but not limited thereto.

The first carrier protein and the second carrier protein may be at least one selected from Ub (ubiquitin), UbR (ubiquitin A77R mutant), Lamp, mODC (mouse ornithine decarboxylase), cmyc, SORT1 (sortilin 1), TRX (Thioredoxin) (for example, TRXwt and/or TRXmt), PSBD (peripheral subunit-binding domain), TRXwt-UbR, TRXwt-Lamp, TRXwt-mODC, TRXwt-cmyc, TRXwt-SORT1, SORT1 w/TM and Lamp w/TM, respectively, but not limited thereto.

The Ub may be degraded well, when the last sequence of Ub, alanine (A) is replaced with arginine (R), in case of being inserted into the N-terminus of the antigen protein, and thus, when the mRNA encoding the carrier protein corresponding to Ub is linked to the 5' end of the mRNA of the antigen protein, mRNA encoding UbR, of which the last sequence is R, may be linked, but not limited thereto.

The TRXwt-UbR, TRXwt-Lamp, TRXwt-mODC and TRXwt-cmyc are proteins in which TRXwt is added to the N-terminus of the UbR, Lamp, mODC, cmyc proteins.

The SORT1 w/TM and Lamp w/TM are proteins in which a transmembrane domain is added to each cytosolic domain of the SORT1 and Lamp.

In the conjugate,
(1) the first carrier protein may be TRXmt, and the second carrier protein may be PSBD;
(2) the first carrier protein may be UbR, and the second carrier protein may be mODC;
(3) the first carrier protein may be UbR, and the second carrier protein may be SORT1;
(4) the first carrier protein may be Lamp, and the second carrier protein may be mODC;
(5) the first carrier protein may be Lamp, and the second carrier protein may be SORT1;
(6) the first carrier protein may be UbR, and the second carrier protein may be cmyc;
(7) the first carrier protein may be UbR, and the second carrier protein may be Lamp;
(8) the first carrier protein may be mODC, and the second carrier protein may be SORT1;
(9) the first carrier protein may be cmyc, and the second carrier protein may be mODC;
(10) the first carrier protein may be cmyc, and the second carrier protein may be SORT1;
(11) the first carrier protein may be Lamp, and the second carrier protein may be Ub;
(12) the first carrier protein may be Lamp, and the second carrier protein may be cmyc;
(13) the first carrier protein may be SORT1, and the second carrier protein may be mODC;
(14) the first carrier protein may be mODC, and the second carrier protein may be Ub;
(15) the first carrier protein may be mODC, and the second carrier protein may be cmyc;
(16) the first carrier protein may be mODC, and the second carrier protein may be Lamp;
(17) the first carrier protein may be cmyc, and the second carrier protein may be Ub;
(18) the first carrier protein may be cmyc, and the second carrier protein may be Lamp;
(19) the first carrier protein may be SORT1, and the second carrier protein may be Ub;
(20) the first carrier protein may be SORT1, and the second carrier protein may be cmyc;
(21) the first carrier protein may be SORT1, and the second carrier protein may be Lamp;
(22) the first carrier protein may be TRXwt, and the second carrier protein may be cmyc;
(23) the first carrier protein may be TRXwt, and the second carrier protein may be Lamp;
(25) the first carrier protein may be TRXwt, and the second carrier protein may be mODC;
(26) the first carrier protein may be TRXwt, and the second carrier protein may be SORT1;
(27) the first carrier protein may be TRXwt, and the second carrier protein may be Ub;
(28) the first carrier protein may be cmyc, and the second carrier protein may be TRXwt;
(29) the first carrier protein may be Lamp, and the second carrier protein may be TRXwt;
(30) the first carrier protein may be mODC, and the second carrier protein may be TRXwt;
(31) the first carrier protein may be SORT1, and the second carrier protein may be TRXwt;
(32) the first carrier protein may be UbR, and the second carrier protein may be TRXwt;
(33) the first carrier protein may be TRXwt-UbR, and the second carrier protein may be SORT1;
(34) the first carrier protein may be TRXwt-Lamp, and the second carrier protein may be mODC;
(35) the first carrier protein may be TRXwt-mODC, and the second carrier protein may be SORT1;
(36) the first carrier protein may be TRXwt-mODC, and the second carrier protein may be Ub;
(37) the first carrier protein may be TRXwt-mODC, and the second carrier protein may be Lamp;
(38) the first carrier protein may be TRXwt-cmyc, and the second carrier protein may be Ub;
(39) the first carrier protein may be TRXwt-SORT1, and the second carrier protein may be Ub;
(40) the first carrier protein may be TRXwt-SORT1, and the second carrier protein may be Lamp;
(41) the first carrier protein may be TRXwt, and the second carrier protein may be SORT1 w/ TM; or
(42) the first carrier protein may be Lamp w/ TM, and the second carrier protein may be TRXwt, but not limited thereto.

In another embodiment, the first carrier protein and the second carrier protein in the conjugate may be exemplified as follows:

**[Table 1]**

| CP_set | N-term (First carrier protein) | C-term (Second carrier protein) |
|---|---|---|
| CPO1 | TRXmt | PSBD |
| CP11 | UbR | mODC |
| CP12 | UbR | SORT1 |
| CP13 | Lamp | mODC |
| CP14 | Lamp | SORT1 |
| CP21 | UbR | cmyc |
| CP22 | UbR | Lamp |
| CP23 | mODC | SORT1 |
| CP24 | cmyc | mODC |
| CP25 | cmyc | SORT1 |
| CP26 | Lamp | Ub |
| CP27 | Lamp | cmyc |
| CP28 | SORT1 | mODC |
| CP31 | mODC | Ub |
| CP32 | mODC | cmyc |
| CP33 | mODC | Lamp |
| CP34 | cmyc | Ub |
| CP35 | cmyc | Lamp |
| CP36 | SORT1 | Ub |
| CP37 | SORT1 | cmyc |
| CP38 | SORT1 | Lamp |
| CP41 | TRXwt | cmyc |
| CP42 | TRXwt | Lamp |
| CP43 | TRXwt | mODC |
| CP44 | TRXwt | SORT1 |
| CP45 | TRXwt | Ub |
| CP46 | cmyc | TRXwt |
| CP47 | Lamp | TRXwt |
| CP48 | mODC | TRXwt |
| CP49 | SORT1 | TRXwt |
| CP50 | UbR | TRXwt |
| CP112 | TRXwt-UbR | SORT1 |
| CP113 | TRXwt-Lamp | mODC |
| CP123 | TRXwt-mODC | SORT1 |
| CP131 | TRXwt-mODC | Ub |
| CP133 | TRXwt-mODC | Lamp |
| CP134 | TRXwt-cmyc | Ub |
| CP136 | TRXwt-SORT1 | Ub |
| CP138 | TRXwt-SORT1 | Lamp |
| CP201 | TRXwt | SORT1 w/ TM |
| CP202 | Lamp w/ TM | TRXwt |

The UbR may comprise the amino acid sequence of SEQ ID NO: 1 or an amino acid sequence having sequence homology of 50% or more, 60% or more, 70% or more, 80% or more, 85% or more, 90% or more, 95% or more or 99% or more to the amino acid sequence, but not limited thereto. A gene encoding the UbR may have the base sequence of SEQ ID NO: 21 or sequence homology of 50% or more, 60% or more, 70% or more, 80% or more, 85% or more, 90% or more, 95% or more or 99% or more to the base sequence, but not limited thereto.

The Ub may comprise the amino acid sequence of SEQ ID NO: 2 or an amino acid sequence having sequence homology of 50% or more, 60% or more, 70% or more, 80% or more, 85% or more, 90% or more, 95% or more or 99% or more to the amino acid sequence, but not limited thereto. A gene encoding the Ub may have the base sequence of SEQ ID NO: 22 or sequence homology of 50% or more, 60% or more, 70% or more, 80% or more, 85% or more, 90% or more, 95% or more or 99% or more to the base sequence, but not limited thereto.

The Lamp may comprise the amino acid sequence of SEQ ID NO: 3 or 4 or an amino acid sequence having sequence homology of 50% or more, 60% or more, 70% or more, 80% or more, 85% or more, 90% or more, 95% or more or 99% or more to the amino acid sequence, but not limited thereto. A gene encoding the Lamp may have the base sequence of SEQ ID NO: 23 or 24 or sequence homology of 50% or more, 60% or more, 70% or more, 80% or more, 85% or more, 90% or more, 95% or more or 99% or more to the base sequence, but not limited thereto. When the Lamp is the first carrier protein, it may have SEQ ID NO: 3 or an amino acid sequence having the above sequence homology, and when it is the second carrier protein, it may have SEQ ID NO: 4 or an amino acid sequence having the above homology, but not limited thereto.

The mODC may comprise the amino acid sequence of SEQ ID NO: 5 or 6 or an amino acid sequence having sequence homology of 50% or more, 60% or more, 70% or more, 80% or more, 85% or more, 90% or more, 95% or more or 99% or more to the amino acid sequence, but not limited thereto. A gene encoding the mODC may have the base sequence of SEQ ID NO: 25 or 26 or sequence homology of 50% or more, 60% or more, 70% or more, 80% or more, 85% or more, 90% or more, 95% or more or 99% or more to the base sequence, but not limited thereto. When the mODC is the first carrier protein, it may have SEQ ID NO: 5 or an amino acid sequence having the above sequence homology, and when it is the second carrier protein, it may have SEQ ID NO: 6 or an amino acid sequence having the above homology, but not limited thereto.

The cmyc may comprise the amino acid sequence of SEQ ID NO: 7 or 8 or an amino acid sequence having sequence homology of 50% or more, 60% or more, 70% or more, 80% or more, 85% or more, 90% or more, 95% or more or 99% or more to the amino acid sequence, but not limited thereto. A gene encoding the cmyc may have the base sequence of SEQ ID NO: 27 or 28 or sequence homology of 50% or more, 60% or more, 70% or more, 80% or more, 85% or more, 90% or more, 95% or more or 99% or more to the base sequence, but not limited thereto. When the cmyc is the first carrier protein, it may have SEQ ID NO: 7 or an amino acid sequence having the above sequence homology, and when it is the second carrier protein, it may have SEQ ID NO: 8 or an amino acid sequence having the above homology, but not limited thereto.

The SORT1 may comprise the amino acid sequence of SEQ ID NO: 9 or 10 or an amino acid sequence having sequence homology of 50% or more, 60% or more, 70% or more, 80% or more, 85% or more, 90% or more, 95% or more or 99% or more to the amino acid sequence, but not limited thereto. A gene encoding the SORT1 may have the base sequence of SEQ ID NO: 29 or 30 or sequence homology of 50% or more, 60% or more, 70% or more, 80% or more, 85% or more, 90% or more, 95% or more or 99% or more to the base sequence, but not limited thereto. When the SORT1 is the first carrier protein, it may have SEQ ID NO: 9 or an amino acid sequence having the above sequence homology, and when it is the second carrier protein, it may have SEQ ID NO: 10 or an amino acid sequence having the above homology, but not limited thereto.

The TRX (specifically, TRXwt) may comprise the amino acid sequence of SEQ ID NO: 11 or 12 or an amino acid sequence having sequence homology of 50% or more, 60% or more, 70% or more, 80% or more, 85% or more, 90% or more, 95% or more or 99% or more to the amino acid sequence, but not limited thereto. A gene encoding the TRX (specifically, TRXwt) may have the base sequence of SEQ ID NO: 31 or 32 or sequence homology of 50% or more, 60% or more, 70% or more, 80% or more, 85% or more, 90% or more, 95% or more or 99% or more to the base sequence, but not limited thereto. When the TRX (specifically, TRXwt) is the first carrier protein, it may have SEQ ID NO: 11 or an amino acid sequence having the above sequence homology, and when it is the second carrier protein, it may have SEQ ID NO: 12 or an amino acid sequence having the above homology, but not limited thereto. The TRXwt refers to a wild type of TRX, which can be obtained from nature without mutation.

The PSBD may comprise the amino acid sequence of SEQ ID NO: 13 or an amino acid sequence having sequence homology of 50% or more, 60% or more, 70% or more, 80% or more, 85% or more, 90% or more, 95% or more or 99% or more to the amino acid sequence, but not limited thereto. A gene encoding the PSBD may have the base sequence of SEQ ID NO: 33 or sequence homology of 50% or more, 60% or more, 70% or more, 80% or more, 85% or more, 90% or more, 95% or more or 99% or more to the base sequence, but not limited thereto.

The TRXwt-UbR may comprise the amino acid sequence of SEQ ID NO: 14 or an amino acid sequence having sequence homology of 50% or more, 60% or more, 70% or more, 80% or more, 85% or more, 90% or more, 95% or more or 99% or more to the amino acid sequence, but not limited thereto. A gene encoding the TRXwt-UbR may have the base sequence of SEQ ID NO: 34 or sequence homology of 50% or more, 60% or more, 70% or more, 80% or more, 85% or more, 90% or more, 95% or more or 99% or more to the base sequence, but not limited thereto.

The TRXwt-Lamp may comprise the amino acid sequence of SEQ ID NO: 15 or an amino acid sequence having sequence homology of 50% or more, 60% or more, 70% or more, 80% or more, 85% or more, 90% or more, 95% or more or 99% or more to the amino acid sequence, but not limited thereto. A gene encoding the TRXwt-Lamp may have the base sequence of SEQ ID NO: 35 or sequence homology of 50% or more, 60% or more, 70% or more, 80% or more, 85% or more, 90% or more, 95% or more or 99% or more to the base sequence, but not limited thereto.

The TRXwt-mODC may comprise the amino acid sequence of SEQ ID NO: 16 or an amino acid sequence having sequence homology of 50% or more, 60% or more, 70% or more, 80% or more, 85% or more, 90% or more, 95% or more or 99% or more to the amino acid sequence, but not limited thereto. A gene encoding the TRXwt-mODC may have the base sequence of SEQ ID NO: 36 or sequence homology of 50% or more, 60% or more, 70% or more, 80% or more, 85% or more, 90% or more, 95% or more or 99% or more to the base sequence, but not limited thereto.

The TRXwt-cmyc may comprise the amino acid sequence of SEQ ID NO: 17 or an amino acid sequence having sequence homology of 50% or more, 60% or more, 70% or more, 80% or more, 85% or more, 90% or more, 95% or more or 99% or more to the amino acid sequence, but not limited thereto. A gene encoding the TRXwt-cmyc may have the base sequence of SEQ ID NO: 37 or sequence homology of 50% or more, 60% or more, 70% or more, 80% or more, 85% or more, 90% or more, 95% or more or 99% or more to the base sequence, but not limited thereto.

The TRXwt-SORT1 may comprise the amino acid sequence of SEQ ID NO: 18 or an amino acid sequence having sequence homology of 50% or more, 60% or more, 70% or more, 80% or more, 85% or more, 90% or more, 95% or more or 99% or more to the amino acid sequence, but not limited thereto. A gene encoding the TRXwt-SORT1 may have the base sequence of SEQ ID NO: 38 or sequence homology of 50% or more, 60% or more, 70% or more, 80% or more, 85% or more, 90% or more, 95% or more or 99% or more to the base sequence, but not limited thereto.

The SORT1 w/ TM may comprise the amino acid sequence of SEQ ID NO: 19 or an amino acid sequence having sequence homology of 50% or more, 60% or more, 70% or more, 80% or more, 85% or more, 90% or more, 95% or more or 99% or more to the amino acid sequence, but not limited thereto. A gene encoding the SORT1 w/ TM may have the base sequence of SEQ ID NO: 39 or sequence homology of 50% or more, 60% or more, 70% or more, 80% or more, 85% or more, 90% or more, 95% or more or 99% or more to the base sequence, but not limited thereto.

The Lamp w/ TM may comprise the amino acid sequence of SEQ ID NO: 20 or an amino acid sequence having sequence homology of 50% or more, 60% or more, 70% or more, 80% or more, 85% or more, 90% or more, 95% or more or 99% or more to the amino acid sequence, but not limited thereto. A gene encoding the Lamp w/ TM may have the base sequence of SEQ ID NO: 40 or sequence homology of 50% or more, 60% or more, 70% or more, 80% or more, 85% or more, 90% or more, 95% or more or 99% or more to the base sequence, but not limited thereto.

The TRXmt may comprise the amino acid sequence of SEQ ID NO: 145 or an amino acid sequence having sequence homology of 50% or more, 60% or more, 70% or more, 80% or more, 85% or more, 90% or more, 95% or more or 99% or more to the amino acid sequence, but not limited thereto. A gene encoding the TRXmt may have the base sequence of SEQ ID NO: 146 or sequence homology of 50% or more, 60% or more, 70% or more, 80% or more, 85% or more, 90% or more, 95% or more or 99% or more to the base sequence, but not limited thereto.

The TRXmt may comprise a sequence in which at least one amino acid selected from the group consisting of amino acids corresponding to the 32th, 35th, 62th, 69th and 73th positions from the N-terminus of the TRX amino acid sequence (specifically, amino acid sequence of SEQ ID NO: 11) is replaced with serine (S), but not limited thereto. The replacement may be that cysteine is replaced with serine.

The protein or polypeptide provided in the present description may be isolated and/or purified from nature, or recombinantly or chemically synthesized. When the amino acid sequence of the protein or polypeptide provided in the present description comprises methionine (Met, M) as the first amino acid residue from the N-terminus, the protein or polypeptide may be recombinantly produced, and methionine at the first amino acid position from the N-terminus may be encoded by a start codon. Therefore, when the amino acid sequence of the protein or polypeptide provided in the present description comprises methionine by recombinant production at the N-terminus, it may be interpreted as comprising an amino acid sequence starting from the second amino acid residue excluding methionine at the first position of the N-terminus by the recombinant production, in case that the protein or polypeptide is obtained by another method (for example, chemical synthesis or isolation from nature) or is not located at the N-terminus in the conjugate.

The mRNA encoding the first carrier protein is linked to the mRNA 5' end of the antigen protein (corresponding to the N-terminus of the antigen protein), and the mRNA encoding the first carrier protein may be linked by adding a base sequence encoding methionine (Met, M) at the 5' end, and for example, "atg" sequence may be added and linked, but not limited thereto.

### Antigen protein

In the conjugate, comprising mRNA of an antigen protein;
mRNA encoding a first carrier protein linked to the 5' end of the mRNA of the antigen protein; and
mRNA encoding a second carrier protein linked to the 3' end of the mRNA of the antigen protein, provided in the present description, the antigen protein may comprise a T cell epitope, but not limited thereto. Specifically, the antigen protein may comprise MHC class I and/or II binding motifs. For example, the antigen protein may comprise the MHC class II binding motif, and may comprise a CD4+ T cell epitope, which is a peptide sequence that can be presented on the surface of the antigen-presenting cells by the MHC class II molecule. The antigen protein may comprise the MHC class I binding motif, and may comprise a CD8+ T cell epitope, which is a peptide sequence that can be presented on the cell surface by the MHC class I molecule. The antigen protein may also comprise both the CD4+ T cell epitope and CD8+ T cell epitope.

In the present description, "MHC (major histocompatibility complex)" or "major histocompatibility complex" is a protein that plays a role of providing an antigen fragment into an immunocyte so as to distinguish non-self molecules, and there are two types of MHC class I and MHC class II, and whereas MHC class I is present in all cells having a nucleus, MHC class II is found in antigen-presenting cells. MHC class I molecules interact with CD8+ cytotoxic T cells, and play an important role in rejection reaction of organ transplant or destroying infected cells. MHC class II molecules play an important role in causing cell-mediated immunity by recognizing non-self antigens through interaction with CD4+ helper T cells.

In general, in an adaptive immune response, when an antigen enters the body, antigen-presenting cells ingest it and decompose it into short peptide fragments, and peptides can bind to MHC class I or MHC class II molecules and be transported to the cell surface. In this way, when the peptides of the antigen bind to MHC class I or MHC class II and are presented on the cell surface of the antigen-presenting cells, T cells recognize them by a T cell receptor (TCR) and are activated and initiate an immune response. In this respect, the antigen protein herein may correspond to an epitope of T cells.

The antigen protein (for example, T cell epitope) may be derived from a tumor antigen, an antigen of an infection source, an autoantigen, or an allergy causing antigen, but not limited thereto. Specifically, the antigen protein (for example, T cell epitope) is predicted to bind to the MHC class I or MHC class II molecules, but may comprise a part of a known tumor antigen, an antigen of an infection source, an autoantigen, or an allergy causing antigen. The antigens may be CD8+ or CD4+ T cell epitopes, but not limited thereto.

The antigen protein (for example, T cell epitope) may comprise a natural or non-natural amino acid sequence capable of inducing an immune response, for example, a T cell or B cell response, in an object, an amino acid having modification after translation, or a peptide mimetic. For example, the antigen protein (for example, T cell epitope) may be about 5 to about 100, or about 5 to about 50 amino acids, or may be about 7 to 35 amino acids, for example, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35 amino acids, but not limited thereto.

In the present description, "tumor antigen" may be interchangeably used with "cancer antigen" , and refers to an antigen expressing in tumor (cancer), causing an immune response. This immune response may accompany antibody generation, or activation of a specific immune-competent cell, or both of them.

The tumor antigen may be derived from an organism with tumor, entire killed or non-activated tumor cells, or lysates, and includes any antigen derived from tumor. The lysates refer to substances generated from application of a process of causing splitting of a normal structure of a cell. In addition, the tumor antigen includes any protein or other substance having antigen characteristics, which is comprised in tumor cells and is expressed differently from normal cells.

The tumor antigen may comprise a tumor-associated antigen (TAA), a tumor-specific antigen (TSA) or a tumor-derived neoantigen, but not limited thereto.

The tumor-associated antigen (TAA) is an antigen which appears more frequently in cancer cells than normal cells or appears at a differentiation stage different from normal cells, and is a tumor shared antigen that is present in a trace amount also in normal cells. Therefore, there is a high possibility that an immune response using this may be nullified by auto-tolerance, which is an immunosuppressive mechanism to prevent damage of self cells, or, on the contrary, an undesired organ may be attacked due to autoimmunity.

The tumor-associated antigen (TAA) may be mTrp2, CT (Cancer-testis) antigen, EGFR, Ova, Melan-A, PSMA (Prostate Specific Membrane Antigen), Survivin, ADAbp (adenosine deaminase-binding protein), cyclophilin b, gp100, CRC (Colorectal associated antigen)-C017-1 A/GA733, CEA (carcinoembryonic antigen), CAP-1, CAP-2, etv6, AML1, PSA (Prostate Specific Antigen), PSA-1, PSA-2, PSA-3, MAGE (melanoma antigen E) (for example, MAGE-A, MAGE-A3, etc.), GAGE (G antigen), BAGE (B melanoma antigen), RAGE (renal tumor antigen), LAGE (L antigen), NAG, GnT-V, MUM-1, CDK4, p53, tyrosinase, Muc1 (mucin 1), HER2/neu, p21ras, N-RAS, K-RAS, RCAS1, α-fetoprotein, E-cadherin, α-catenin, β-catenin, γ-catenin, p120ctn, PRAME, NY-ESO-1, TRP2, Mammaglobin-A, metallopanstimulin-1 (MPS-1), cytochrome P450 isoform 1B1, 90K/Mac-2 binding protein, Ep-CAM (MK-1), HSP-70, hTERT (TRT), LEA, TAGE-1, 5T4, gp70, SCP-1, cmyc, cyclin B1, MDM2, p62, Koc, IMP1, TA90, OA1, CT-7, HOM-MEL-40/SSX-2, SSX-1, SSX-4, HOM-TES-14/SCP-1, HOM-TES-85, HDAC5, MBD2, TRIP4, NY-CO-45, KNSL6, HIP1R, Seb4D, KIAA1416, IMP1, 90K/Mac-2 binding protein, MDM2, or LMNA, but not limited thereto.

The tumor-specific antigen (TSA) refers to an antigen specifically present only in cancer cells, and specifically, may be M12, M20, M21, M30 or M44, but not limited thereto. In particular, when tumor is proliferated in cancer patients, cancer cell-specific gene mutation occurs, generating a new antigen epitope capable of stimulating T cells, and this is called a neoantigen. In other words, the neoantigen may comprise a cancer cell-specific gene mutation, and is selectively expressed only in cancer cells differently from tumor shared antigens expressed in a trace amount also in normal cells, and thus, it is recognized as a non-self foreign epitope by the autoimmune system, and induces strong anti-cancer immune activity.

When peptides generated from mutated DNA are displayed on the MHC on the cell surface, a T cell receptor (TCR) recognizes it, but mutation does not occur in normal cells or tissues, so neoantigen-specific T cells are free from self-tolerance or autoimmune problems. Due to such advantages, the neoantigen is considered an ideal target for T cell-based cancer immunotherapy.

Causes of generating neoantigens include frame-shift (frame-shift deletion or insertion) in which translation of genetic codes is dislocated as one or more nucleotides consisting of DNA are added or deleted, point mutation occurring by replacing one nucleotide with another one, other missense mutation, splice-site mutation, read-through mutation, or gene-fusion mutation or the like, but are not limited thereto.

Neoantigens are predicted through specific cancer cell genome analysis of an individual cancer patient, and as one example, cancer cells are obtained from a patient' s tumor, and DNA is extracted, and then a base sequence is analyzed, and this is compared and analyzed with a base sequence of a normal cell, and then among base sequences of various mutation-occurred portions, neoantigens that stimulate T cells may be identified. For this, processing of big data such as next generation sequencing (NGS), whole-exome sequencing (WES), or RNA sequencing, a computer program for predicting MHC binding, or artificial intelligence (AI) for prediction of neoantigens, or the like, may be used, but not limited thereto. Since mutant forms are not shared between patients, neoantigens can be produced as a personalized cancer vaccine.

The tumor-derived neoantigen may comprise mutation expressed specifically in cancer cells, but not limited thereto.

The tumor antigen may comprise the amino acid sequence of SEQ ID NO: 51, 53, 55, 57 or 59, or an amino acid sequence having sequence homology of 50% or more, 60% or more, 70% or more, 80% or more, 85% or more, 90% or more, 95% or more or 99% or more to the amino acid sequence, but not limited thereto. A gene encoding the tumor antigen may have the base sequence of SEQ ID NO: 52, 54, 56, 58 or 60, or sequence homology of 50% or more, 60% or more, 70% or more, 80% or more, 85% or more, 90% or more, 95% or more or 99% or more to the base sequence, but not limited thereto.

The antigen of the infection source may be an antigen derived from a virus, bacterium, parasite, or fungus, but not limited thereto.

The virus-derived antigen may be an antigen derived from a poxvirus, Variola virus, Ebola virus, Marburg virus, dengue virus, influenza virus, parainfluenza virus, respiratory syncytial virus, measle virus, human immunodeficiency virus, human papillomavirus, varicella-zoster virus, herpes simplex virus, cytomegalovirus, Epstein-Barr virus, JC virus, rhabdovirus, rotavirus, rhinovirus, adenovirus, papillomavirus, parvovirus, picornavirus, poliovirus, mumps-causing virus, rabies-causing virus, reovirus, rubella virus, togavirus, orthomyxovirus, retrovirus, hepadnavirus, coxsackievirus, equine encephalitis virus, Japanese encephalitis virus, yellow fever virus, Rift Valley fever virus, hepatitis A virus, hepatitis B virus, hepatitis C virus, hepatitis D virus or hepatitis E virus, but not limited thereto.

The bacteria-derived antigen may be an antigen derived from Borrelia species, Bacillus anthracis, burgdorferi, Bordetella pertussis, Campylobacter jejuni, Chlamydia species, Chlamydia psittaci, Chlamydial trachomatis, Clostridium species, Clostridium tetani, Clostridium botulinum, Clostridium perfringens, Corynebacterium diphtheriae, Coxiella species, Enterococcus species, Erlichia species, Escherichia coli, Francisella tularensis, Haemophilus species, Haemophilus influenzae, Haemophilus parainfluenzae, Lactobacillus species, Legionella species, Legionella pneumophila, Leptospirosis interrogans, Listeria species, Listeria monocytogenes, Mycobacterium species, Mycobacterium tuberculosis, Mycobacterium leprae, Mycoplasma species, Mycoplasmapneumoniae, Neisseria species, Neisseria meningitidis, Neisseria gonorrhoeae, Pneumococcus species, Pseudomonas species, Pseudomonas aeruginosa, Salmonella species, Salmonella typhi, Salmonella enterica, Rickettsia species, Rickettsia rickettsii, Rickettsia typhi, Shigella species, Staphylococcus species, Staphylococcus aureus, Streptococcus species, Streptococccus pnuemoniae, Streptococcus pyrogenes, Streptococcus mutans, Treponema species, Treponema pallidum, Vibrio species, Vibrio cholerae, or Yersinia pestis, but not limited thereto.

The fungus-derived antigen may be an antigen derived from fungi selected from Candida species, Cryptococcus species, Coccidioides species, Histoplasma species and Aspergillus species, but not limited thereto.

The parasite-derived antigen may be an antigen derived from Plasmodium, Trypanosome, Schistosome or Leishmania, but not limited thereto.

The autoantigen may be an antigen which is known or suspected to cause autoimmunity, but not limited thereto.

The allergy causing antigen (allergen) may be an antigen which is known or suspected to cause allergy, but not limited thereto.

### Use of immunization of conjugate

Other embodiment provides a composition for immunization and/or a vaccine composition comprising the conjugate.

The composition relates to a composition for prevention, improvement and/or treatment of cancer, infectious disease, autoimmune disease and/or allergic disease.

In the present description, "composition for immunization" refers to any composition which can induce an immune response, and "vaccine" refers to a composition for immunization for reducing or preventing a risk of disease or infection, or improving or treating existing disease or infection, by inducing an immune response. The composition for immunization may be a composition for immunization against cancer, infectious disease, autoimmune disease and/or allergic disease.

Specifically, the composition for immunization may mean a composition that can induce an immune response by antibody generation against the antigen.

The composition may be a formulation in a form that comprises the conjugate provided in the present description and can be administered into a subject, to elicit an immune response. The composition may be used conveniently to prevent or improve or treat disease. After introduced into a subject or host, the composition may elicit an immune response, and preferably, may elicit a T-cell-mediated immune response.

The vaccine may be an anticancer vaccine comprising a tumor antigen, for example, a tumor antigen sequence, particularly, a neoantigen sequence, obtained from for example, patient tumor analysis, and thereby, personalized proliferation and activation of anticancer T cells may be triggered to the maximum to increase the anticancer immune effect.

The composition may also comprise an additional adjuvant that enhances effectiveness of the vaccine. The appropriate adjuvant includes (1) aluminum salts (alum), for example, aluminum hydroxide, aluminum phosphate, aluminum sulfate, and the like; (2) oil-in-water emulsion preparations (comprising or not comprising a specific immunostimulant such as a muramyl peptide or bacterial cell wall component), for example, (a) MF59 (WO90/14837) formulated into submicron particles, comprising 5% squalene, 0.5% tween 80 and 0.5% span 85 (not necessarily, but selectively, comprising N-acetylmuramyl-L-alanyl-D-isoglutaminyl-L-alanine-2-(1' ,2' -dipalmitoyl-sn-glycero-3-hydroxyphosphoryloxy)-ethylamine (MTP-PE) in various amounts), (b) SAF microfluized into submicron particles or shaken to generate emulsion in a large particle size, comprising 10% squalene, 0.4% tween 80, 5% pluronic-blocked polymer, and N-acetyl-muramyl-L-threonyl-D-isoglutamine (thr-MDP), and (c) RibiTM adjuvant system (RAS) comprising at least one bacterial cell wall component selected from the group consisting of monophosphoryllipid A (MPL), trehalose dimycolate (TDM), and cell wall skeleton, 2% squalane, and 0.2% tween 80; (3) saponin adjuvants; (4) complete Freund' s adjuvants and incomplete Freund' s adjuvants (IFA); (5) cytokines, for example, interleukins (for example, IL-1, IL-2, IL-4, IL-5, IL-6, IL-7, IL-12, etc.), interferons (for example, gamma interferon), macrophage colony stimulating factors (M-CSF), tumor necrosis factors (TNF), and the like; (6) detoxified mutants of bacterial ADP-ribosylating toxins, for example, cholera toxins (CT), pertussis toxins (PT), or heat labile toxins of E. coli (LT), particularly, LT-R72, CT-S109, PT-K9/G129 (WO93/13302 and WO92/19265); and (7) other substances that act as an adjuvant enhancing effectiveness of a vaccine, but not limited thereto.

The composition may further comprise a pharmaceutically acceptable carrier, diluent and/or excipient in a commonly used amount if needed.

The pharmaceutically acceptable carrier is commonly used for preparation, and includes lactose, dextrose, sucrose, sorbitol, mannitol, starch, acacia gum, calcium phosphate, alginate, gelatin, calcium silicate, microcrystalline cellulose, polyvinyl pyrrolidone, cellulose, water, syrup, methyl cellulose, methylhydroxy benzoate, propylhydroxy benzoate, talc, magnesium stearate, and mineral oil and the like, but not limited thereto. The composition may further comprise a lubricant, a wetting agent, a sweetener, a flavoring agent, an emulsifier, a suspending agent, a preservative, and the like, in addition to the above components.

The conjugate and/or composition comprising the same as an active ingredient may be prepared in a unit dose form, or be prepared as contained in a multi-dose container, by preparation using a pharmaceutically acceptable carrier and/or excipient, according to a method that those skilled in the art can easily implement.

For example, the composition may comprise common salt water suspended or dissolved, or buffered aqueous solution media. For example, commonly, a diluent, for example, water, salt water, glycerol, ethanol, and the like may be comprised, and auxiliary substances, for example, wetting agents, emulsifiers, pH buffers, and the like may be present in the composition.

Forms suitable for injection include sterile aqueous solution (aqueous) or dispersion and sterile powders for instant preparation of sterile injection solution or dispersion. They should be stable under conditions of preparation and should be preserved against contamination by microorganisms such as bacteria or fungi. Microbial contamination may be prevented using various antibacterial agents and antifungal agents, for example, paraben, chlorobutanol, phenol, sorbic acid, thimerosal, and the like. In many cases, it is preferable to comprise an isotonic agent, such as sugar or sodium chloride. Prolonged absorption of an injection composition may be achieved by using an agent that delays absorption, for example, aluminum monostearate or gelatin in the composition.

In the afore-mentioned solvent, the required amount of conjugate and other various components listed above are combined, and if needed, the other components excluding the conjugate and/or heat-sensitive adjuvant cytokine and the like, for example, buffer solution such as PBS are filtered and sterilized, thereby preparing a sterile injection solution. In general, from basic dispersion media and those described above, various sterilized active ingredients are incorporated into a sterile vehicle comprising other components required, thereby preparing dispersion. In case of sterile powders for preparing the sterile injection solution, preferable preparation methods are vacuum drying and freeze-drying methods. By these methods, powders of the active ingredients, and any desired ingredients from the sterile-filtered solution thereof described above are obtained.

Patients subject to administration of the conjugate or a composition comprising the same as an active ingredient may be mammals, for example, humans, primates including monkeys, etc., rodents including rats, mice, etc., and the like, but not limited thereto.

Another aspect of the present invention relates to a method for causing, inducing, or promoting an immune response against an antigen, in humans, for example, cancer patients, and the method comprises administering an effective dose of the composition or conjugate into patients, for example, cancer patients.

In the present description, "immune response" is the result of stimulation directly or indirectly through cellular or cytokine mediation, and refers to changes in activity of cells of the immune system, for example, B cells, T cells or monocytes. The immune response may be specific (T cell and/or B cell) and/or non-specific immune responses.

Without limiting the action of the present invention in any way, delivering the conjugate according to the present invention induces an immune response, and in particular, it is especially useful for inducing a T cell response, for example, a CD4+ T cell response or CD8+ T cell response against an antigen. The CD4+ T cell response and CD8+ T cell response may occur with or independently of a humoral response or other specific or non-specific immune responses.

Other aspect of the present invention relates to using the conjugate in relation to treatment and/or prevention of disease states. Examples of diseases that can be treated according to the method of the present invention include various kinds of cancer diseases, infectious diseases, autoimmune diseases and allergic diseases.

The cancer may be solid cancer or blood cancer, and specifically, may be breast cancer, lung cancer, prostate cancer, ovarian cancer, brain cancer, liver cancer, cervical cancer, endometrial cancer, uterine cancer, colon cancer, large intestine cancer, colorectal cancer, rectal cancer, kidney cancer, nephroblastoma, skin cancer, oral squamous carcinoma, epidermoid carcinoma, nasopharyngeal cancer, head and neck cancer, bone cancer, esophageal cancer, bladder cancer, lymphatic cancer (for example, Hodgkin lymphoma or non-Hodgkin lymphoma), gastric cancer, pancreatic cancer, testicular cancer, thyroid cancer, follicular carcinoma, melanoma, myeloma, multiple myeloma, mesothelioma, osteosarcoma, myelodysplastic syndrome, tumor of mesenchymal origin, soft tissue sarcoma, liposarcoma, gastrointestinal stromal sarcoma, malignant peripheral nerve sheath tumor (MPNST), Ewing sarcoma, leiomyosarcoma, mesenchymal chondrosarcoma, lymphosarcoma, fibrosarcoma, rhabdosarcoma, testocarcinoma, neuroblastoma, modulloblastoma, glioma, skin benign tumor, or leukemia. The lung cancer may be, for example, small cell lung cancer (SCLC) or non-small cell lung cancer (NSCLC). The leukemia may be, for example, acute myeloid leukemia (AML), chronic myeloid leukemia (CML), acute lymphoblastic leukemia (ALL) or chronic lymphoblastic leukemia (CLL). The subject to be treated may be a subject receiving secondary anti-hyperproliferative therapy. For example, the secondary anti-hyperproliferative therapy may be chemotherapy, radiotherapy, immunotherapy, phototherapy, cryotherapy, tomotherapy, hormonal therapy, or surgical operation.

Other embodiment provides a method for immunization, comprising administering the conjugate and/or the composition into a subject in need of immunization, and specifically, provides a method for immunization against cancer, by administering it into a subject in need of immunization against cancer.

Other embodiment provides a method for preventing or treating disease, for example, cancer, infectious disease, autoimmune disease, or allergic disease, by enhancing an immune response using the composition. Herein, administration of the composition causes, induces, or diversely promotes an immune response that inhibits, ceases, delays or prevents occurrence or progression.

Direct delivery of the composition may be generally delivering systemically, subcutaneously, intradermally, intraperitoneally, intravascularly (intravenously), intramuscularly, or locally, or be delivering into tissue gaps. The composition may be also administered into lesions. The administration regime may be a single dose or multiple dose schedule.

In the present description, "effective dose" refers to an amount sufficient to achieve a desired result, for example, an amount effective in treating or preventing cancer, when administered into a subject including humans. The effective dose may vary depending on factors such as the individual' s disease condition, age, gender and body weight and the like. The dosage or treatment regime may be adjusted to provide an optimal therapeutic response as understood by those skilled in the art.

The therapeutic regime of a subject using a therapeutically effective dose may consist of a single administration, or as another example, comprise a series of applications. The duration of the treatment phase depends on various factors such as the severity of disease, the age of the subject, the concentration of the vaccine, the reactivity to the vaccine of the patient or combinations thereof. In addition, an effective dose of a vaccine used for treatment may be understood to be increased or decreased during the course of an individual treatment regimen. Variations in dose may occur, and it may be clarified through standard diagnostic assays known in the art. The vaccine, for example, cancer vaccine, of the present invention may be administered, before treatment, during treatment, or after treatment, which uses common anticancer agents, radiation therapy, hormone therapy, biotherapy and/or surgical tumor excision.

### [ADVANTAGEOUS EFFECTS]

The present invention relates to a conjugate comprising mRNA of an antigen protein, and mRNA encoding a carrier protein linked to the 5' end and/or 3' end of the mRNA of the antigen protein, and a composition for immunization and/or a vaccine composition comprising thereof, and has an effect of stably increasing expression of the antigen protein.

### [BRIEF DESCRIPTION OF THE DRAWINGS]

FIGs. 1 and 2 showed vector maps of pUC57-T7-MP-A118 and pEGFP used for construction of mRNA to be used for carrier protein screening, respectively.
FIG. 3 showed a schematic diagram of the carrier protein vector used for construction of mRNA to be used for carrier protein screening.
FIG. 4 showed the vector map of the pUC57-T7-CP12G carrier protein vector.
FIG. 5 showed the result of confirming delivery of CD4/CD8 antigens to the processing/presentation pathway, respectively, in case of treatment of a proteasome inhibitor and a lysosomal inhibitor for carrier protein primary screening.
FIG. 6 showed a schematic diagram of cell expression in case that a transmembrane domain is added to a cytosolic domain of SORT1 and Lamp.
FIG. 7 showed the result of confirming delivery of CD4/CD8 antigens to the processing/presentation pathway, respectively, in case of treatment of a proteasome inhibitor and a lysosomal inhibitor for secondary screening of the carrier protein.
FIG. 8 showed a schematic diagram of the vector used for construction of a vector comprising a carrier protein and an epitope.
FIG. 9 showed the vector map of pUC57-T7-5epitopes.
FIG. 10 showed a schematic diagram of the pLRK33-empty empty vector used for construction of a vector comprising a carrier protein and an epitope.
FIG. 11 showed a schematic diagram showing the process of inserting a fragment comprising an epitope, a carrier protein, and the like into an empty vector during the construction process of a vector comprising a carrier protein and an epitope.
FIG. 12 showed the vector map of pLRK33-hTrx_WT.
FIG. 13 showed the vector map of the pLRK33-hTrx_m vector used for constructing a comparative example vector comprising no carrier protein.
FIG. 14 showed the result of confirming the expression level of the epitope in mRNA according to whether the carrier protein is comprised in the K562 cell line.
FIG. 15 showed the result of confirming the expression level of the epitope in mRNA according to whether the carrier protein is comprised in human dendritic cells.
FIG. 16 showed the result of measuring the immunogenicity against the epitope in mRNA according to whether the carrier protein is comprised in human dendritic cells.

### [MODE FOR INVENTION]

Hereinafter, the present invention will be described in more detail by examples. However, the following examples are intended to illustrate the contents of the present invention only, but the scope of the present invention is not limited by the following examples.

### Example 1. Carrier protein screening

### Example 1-1. Construction of mRNA encoding carrier protein

Carrier protein primary screening was conducted through confirmation of whether CD4/CD8 antigens were appropriately delivered to the processing/presentation pathway by mRNA encoding carrier proteins.

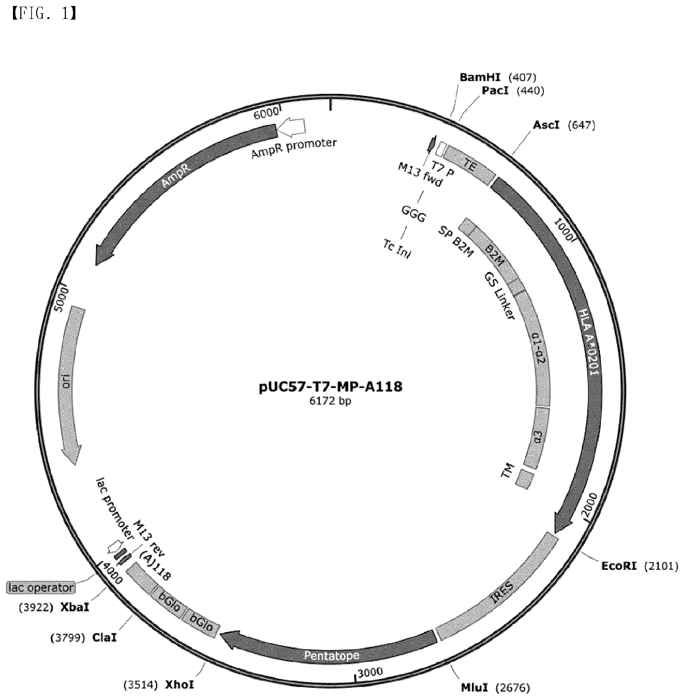

Specifically, for in vitro transcription, pUC57-T7-MP-A118 was constructed by requesting construction of a required vector based on pUC57 vector (Cosmogenetech) to GeneWiz company, and the vector map of the corresponding vector was shown in FIG. 1.

AscI (Enzynomics; R068M) and EcoRI (Enzynomics; R002M) restriction enzymes were treated to the constructed vector, and it was purified with Expin Gel SV (Gene all, Cat#102-102). Through the purification process, among the cleaved vector strands, for the vector strand in which the AscI site was located at the N-terminus and the EcoRI site was located at the C-terminus, to place carrier proteins at each of the N-terminus and C-terminus of the vector by the combinations in Table 2 below, PCR was performed by EZ-fusion HT Cloning kit (Enzynomics; Cat#: EZ015TL) using the T7 oligonucleotides of Table 3 below corresponding to the carrier protein depending on the combinations of Table 2 below and using Platinum^{™} SuperFi II Green PCR Master Mix (ThermoFisher; cat#: 12369050), and the carrier protein was inserted into the AscI site and EcoRI site to construct pUC57-T7-CP01G to pUC57-T7-CP50G plasmids of Table 2 below, and the specific preparation process of each carrier protein was described below:
(i) mODC
   mODC fragment for C-terminal insertion was generated by performing PCR after mixing oligonucleotides oCmODC-BE1-F1, omODC-R2, omODC-F3, and oCmODC-X1-R4 (Table 3). mODC fragment for C-terminal insertion was generated by purifying the previously generated mODC fragment N-terminal insertion, and performing PCR using oligonucleotides oNmODC-A1-F1 and oNmODC-BE1-R2 (Table 3).
(ii) cmyc
   cmyc fragment for N-terminal insertion was generated by performing PCR after mixing oligonucleotides oNcmyc-A1-F1 and oNcmyc-BE1-R2 (Table 3). cmyc fragment for N-terminal insertion was generated by purifying the previously generated cmyc fragment C-terminal insertion, and performing PCR using oligonucleotides oCcmyc-BE1-F1 and oCcmyc-X1-R2 (Table 3).
(iii) Lamp
   Lamp fragment for N-terminal insertion was generated by performing PCR after mixing oligonucleotides oNIi-A1-F1, and oNIi-BE1-R2 (Table 3). Lamp fragment for N-terminal insertion was generated by purifying the previously generated Lamp fragment C-terminal insertion, and performing PCR using oligonucleotides oCli-BE1-F1 and oCIi-X1-R2 (Table 3).
(iv) SORT1
   SORT1 fragment for C-terminal insertion was generated by performing PCR after mixing oligonucleotides oCSORT-BE1-F1, oSORT-R2, oSORT-F3, and oCSORT-X1-R4 (Table 3). SORT1 fragment for C-terminal insertion was generated by purifying the previously generated SORT1 fragment N-terminal insertion, and performing PCR using oligonucleotides oNSORT-A1-F1 and oNSORT-BE1-R2 (Table 3).
(v) TRX
   It was produced by performing PCR after mixing all the oNhTRX-A1-F, oNhTRX-BE1-R, oTRXwt-1F, oTRXwt-2R, oTRXwt-3F, oTRXwt-4R, oTRXwt-5F, oTRXwt-6R, and oTRXwt-3F1 oligonucleotides of Table 3 below.
(vi) PSBD
   It was produced by performing PCR after mixing all the oChPSBD-BE1-F, and oChPSBD-X1-R oligonucleotide of Table 3 below.
(vii) Ub and UbR
   Since alanine (A), the last sequence of Ub inserted at the N-terminus has the characteristic of being easily decomposed when it is replaced with arginine (R), in order that the last sequence is R when inserting Ub at the N-terminus, Ub was inserted by using the oNUbR-BE1-R5 oligonucleotide of Table 3 below (UbR).

Specifically, PCR was performed using the oNUbR-A1-F1, oUbR-R2, oUbR-F3, oNUbR-BE1-R5 of Table 3 below to produce UbR, and Ub was produced and inserted by purifying the UbR PCR product and performing PCR using oCUb-BE1-F1 and oCUb-X1-R2 of Table 3 below.

**[Table 2]**

| Plasmid type | CP_N | CP_C |
|---|---|---|
| pUC57-T7-CP01G | TRXmt (Thioredoxin) | PSBD (peripheral subunit-binding domain) |
| pUC57-T7-CP11G | UbR (ubiquitin A77R mutant) | mODC (mouse ornithine decarboxylase) |
| pUC57-T7-CP12G | UbR | SORT1 (sortilin 1) |
| pUC57-T7-CP13G | Lamp | mODC |
| pUC57-T7-CP14G | Lamp | SORT1 |
| pUC57-T7-CP21G | UbR | cmyc |
| pUC57-T7-CP22G | UbR | Lamp |
| pUC57-T7-CP23G | mODC | SORT1 |
| pUC57-T7-CP24G | cmyc | mODC |
| pUC57-T7-CP25G | cmyc | SORT1 |
| pUC57-T7-CP26G | Lamp | Ub (ubiquitin) |
| pUC57-T7-CP27G | Lamp | cmyc |
| pUC57-T7-CP28G | SORT1 | mODC |
| pUC57-T7-CP31G | mODC | Ub |
| pUC57-T7-CP32G | mODC | cmyc |
| pUC57-T7-CP33G | mODC | Lamp |
| pUC57-T7-CP34G | cmyc | Ub |
| pUC57-T7-CP35G | cmyc | Lamp |
| pUC57-T7-CP36G | SORT1 | Ub |
| pUC57-T7-CP37G | SORT1 | cmyc |
| pUC57-T7-CP38G | SORT1 | Lamp |
| pUC57-T7-CP41G | TRXwt | cmyc |
| pUC57-T7-CP42G | TRXwt | Lamp |
| pUC57-T7-CP43G | TRXwt | mODC |
| pUC57-T7-CP44G | TRXwt | SORT1 |
| pUC57-T7-CP45G | TRXwt | Ub |
| pUC57-T7-CP46G | Cmyc | TRXwt |
| pUC57-T7-CP47G | Lamp | TRXwt |
| pUC57-T7-CP48G | mODC | TRXwt |
| pUC57-T7-CP49G | SORT1 | TRXwt |
| pUC57-T7-CP50G | UbR | TRXwt |
| pUC57-T7-CP112G | TRXwt-UbR | SORT1 |
| pUC57-T7-CP113G | TRXwt-Lamp | mODC |
| pUC57-T7-CP123G | TRXwt -mODC | SORT1 |
| pUC57-T7-CP131G | TRXwt -mODC | Ub |
| pUC57-T7-CP133G | TRXwt -mODC | Lamp |
| pUC57-T7-CP134G | TRXwt-cmyc | Ub |
| pUC57-T7-CP136G | TRXwt-SORT1 | Ub |
| pUC57-T7-CP138G | TRXwt-SORT1 | Lamp |
| pUC57-T7-CP201G | TRXwt | SORT1 w/ TM |
| pUC57-T7-CP202G | Lamp w/ TM | TRXwt |

| | | |
|---|---|---|
| (In Table 2 above, TRXwt refers to the wild type of TRX, that can be obtained from nature without mutation, and TRXmt refers to one in which cysteine (C) corresponding to the 32th, 35th, 62th, 69th and 73th from the N-terminus of the TRX amino acid sequence of SEQ ID NO: 11 is replaced with serine (S)) | | |

**[Table 3]**

| Type | Oligonucleotide name | Sequence (5'->3') | SEQ ID NO: |
|---|---|---|---|
| Ub(R) | oNUbR-A1-F1 | caggcctccacaaccggcgcgccaccatgcagatcttcgtgaaaaccctgacc ggcaagaccatcacactggaagtggaacccagcgaca | 67 |
| | oUbR-R2 | cggcgaagatcagtctctgctggtcaggagggatgccctctttgtcctggatc ttggccttcacgttctcgatggtgtcgctgggttcc | 68 |
| | oUbR-F3 | gagactgatcttcgccggcaagcagctggaagatggcagaaccctgagcgact acaacatccagaaagagtctaccctgcacctggtgct | 69 |
| | oNUbR-BE1-R5 | tccaccagatcctccgcctccggaaccgcctcttccacctctcagtctcagca ccaggtgcagggta | 70 |
| | oCUb-BE1-F1 | ggcggttccggaggcggaggatctggtggacagatcttcgtgaaaacc | 71 |
| | oCUb-X1-R2 | gcaagaaagcgagctctcgagtttatccacctctcagtct | 72 |
| mODC | oCmODC-BE1-F1 | ggcggttccggaggcggaggatctggtggactgatgaagcagatccagagcca cggcttcccacctga | 73 |
| | omODC-R2 | ccggactcttgggcacagctcataggcagggtgccatcgtcctgttcttccac ctcaggtgggaagccgt | 74 |
| | omODC-F3 | tgcccaagagtccggcatggatagacatcctgccgcctgtgccagcgccagaa tcaatgttatgta | 75 |
| | oCmODC-X1-R4 | gcaagaaagcgagctctcgagtttacataacattgattctggcgctggcacag gcggcaggatgtct | 76 |
| | oNmODC-A1-F1 | caggcctccacaaccggcgcgccaccatgctgatgaagcag | 77 |
| | oNmODC-BE1-R2 | tccaccagatcctccgcctccggaaccgcccataacattgattct | 78 |
| cmyc | oNcmyc-A1-F1 | caggcctccacaaccggcgcgccaccatgcacgaggaaacccct | 79 |
| | oNcmyc-BE1-R2 | tccaccagatcctccgcctccggaaccgcccttttccacgctgac | 80 |
| | oCcmyc-BE1-F1 | ggcggttccggaggcggaggatctggtggacacgaggaaacccctccaaccac aagcagcgacagcgaggaagaaca | 81 |
| | oCcmyc-X1-R2 | gcaagaaagcgagctctcgagtttacttttccacgctgacgacgtcgatctct tcctcgtcctcttgttcttcctcgctgtcgctgc | 82 |
| Lamp | oNIi-A1-F1 | caggcctccacaaccggcgcgccaccatggacgaccagagggacctgatcagc aacaacgagcagctgcccatgctcggcagaag | 83 |
| | oNIi-BE1-R2 | tccaccagatcctccgcctccggaaccgcctctgctacatttgctctcaggag cgccaggccttctgccgagcatggg | 84 |
| | oCIi-BE1-F1 | ggcggttccggaggcggaggatctggtggagacgaccagagggac | 85 |
| | oCIi-X1-R2 | gcaagaaagcgagctctcgagtttatctgctacatttgct | 86 |
| | oLAMPwTM-1F | caggcctccacaaccggcgcgccaccatggacgaccagagggacctgatcagc aacaacgagcagctgccca | 87 |
| | oLAMPwTM-2R | agcgccaggccttctgccgagcatgggcagctgctcgtt | 88 |
| | oLAMPwTM-3F | aggcctggcgctcctgagagcaaatgtagcagaggcgctctgtacaccggctt tagcatcctggtcacact | 89 |
| | oLAMPwTM-4R | tccaccagatcctccgcctccggaaccgccgtacagaaagtaggctgtggtag cctgtccggccagcagcagtgtgaccaggatgctaa | 90 |
| Sortilin (SORT1) | oCSORT-BE1-F1 | ggcggttccggaggcggaggatctggtggaaagaaatacgtgtgcggcggcag att | 91 |
| | oSORT-R2 | agggcatcgacaccatcaacaccattggcctcggcgtgctgctgcaggacgct atatctgtgcaccaggaatctgccgccgca | 92 |
| | oSORT-F3 | tggtgtcgatgccctggataccgccagccacacaaacaagagcggctaccacg acgacagcgacgaggatctgcttgagtaa | 93 |
| | oCSORT-X1-R4 | gcaagaaagcgagctctcgagtttactcaagcagatcctcgtcgctgtcgtcg tggtag | 94 |
| | oNSORT-A1-F1 | caggcctccacaaccggcgcgccaccatgaagaaatacgtgtgc | 95 |
| | oNSORT-BE1-R2 | tccaccagatcctccgcctccggaaccgccctcaagcagatcctc | 96 |
| | oSORTwTM-1F | ggcggttccggaggcggaggatctggtggaagcgtgccaatcatcctggccat cgtgggcctgatgctggttaca | 97 |
| | oSORTwTM-2R | cgctatatctgtgcaccaggaatctgccgccgcacacgtatttcttaacgatc agcacgccggcaaccactgtaaccagcatcaggccca | 98 |
| | oSORTwTM-3F | ctggtgcacagatatagcgtcctgcagcagcacgccgaggccaatggtgttga tggtgtcgatgccctggataccgccagccacacaaa | 99 |
| | oSORTwTM-4R | gcaagaaagcgagctctcgagtttactcaagcagatcctcgtcgctgtcgtcg tggtagccgctcttgtttgtgtggctggcggtat | 100 |
| TRX | oNhTRX-A1-F | caggcctccacaaccggcgcgccaccatggtgaagcagatcgag | 101 |
| | oNhTRX-BE1-R | tccaccagatcctccgcctccggaaccgcccaccagctcgttgat | 102 |
| | oTRXwt -1F | caggcctccacaaccggcgcgccaccatggtcaagcagatcgagagcaagacc gccttccaagaggcccttgatgccgctggcgata | 103 |
| | oTRXwt -2R | gcaggggccgcaccaagtggcggaaaaatccaccaccaccagcttatcgccag cggcatc | 104 |
| | oTRXwt-3F | gcggcccctgcaagatgatcaagccattcttccacagcctgagcgagaagta | 105 |
| | oTRXwt-4R | cgcactcagaggccacatcctggcagtcgtccacgtccacttccaggaagatc acgttgctgtacttctcgctcaggctgtggaagaat | 106 |
| | oTRXwt-5F | tggcctctgagtgcgaagtgaagtgcatgcccaccttccagttcttcaagaaa ggccagaaagtgggcgagttcagcggcgccaacaaa | 107 |
| | oTRXwt -6R | tcctccgcttcctccgccgccagatccgccaactagttcgttgatggtggctt ccagcttctctttgttggcgccgctgaa | 108 |
| | oTRXwt-3F1 | cacttggtgcggcccctgcaagatgatcaagccattcttccacagcctgagcg agaagta | 109 |
| PSBD | oChPSBD-BE1-F | ggcggttccggaggcggaggatctggtggagtgatcgccatgcct | 110 |
| | oChPSBD-X1-R | gcaagaaagcgagctctcgagtttaagccagccaagcgtc | 111 |
| EGFP | oEGFP-BE1-F | ggcggttccggaggcggaggatctggtggagtgagcaagggcgag | 112 |
| | oEGFP-BE1-R | tccaccagatcctccgcctccggaaccgcccttgtacagctcgtc | 113 |

| | | | |
|---|---|---|---|
| (In Table 3 above, "tccgga" in bold corresponds to the BspEI site, and the BspEI site was inserted simultaneously with insertion of the carrier protein) | | | |

After insertion of the carrier protein, BspEI (NEB, R0540S) restriction enzyme was treated and it was purified with Expin Gel SV (Gene all, Cat#102-102).

After that, 10 ng of pEGFP vector (Clontech, cat.no. 6077-1, GenBankU76561.1) comprising EGFP reporter as a template was prepared, and 10 pmole of each of the EGFP oligonucleotides (oEGFP-BE1-F and oEGFP-BE1-R) of Table 3, and 100 µl of Platinum^{™} SuperFi II Green PCR Master Mix (ThermoFisher; cat#: 12369050) were prepared, and using them, PCR was performed.

A carrier protein vector in which EGFP was inserted was obtained, by treating BspEI (NEB, R0540S) restriction enzyme after performing PCR, and subcloning into a plasmid capable of expressing each of the purified carrier protein combination.

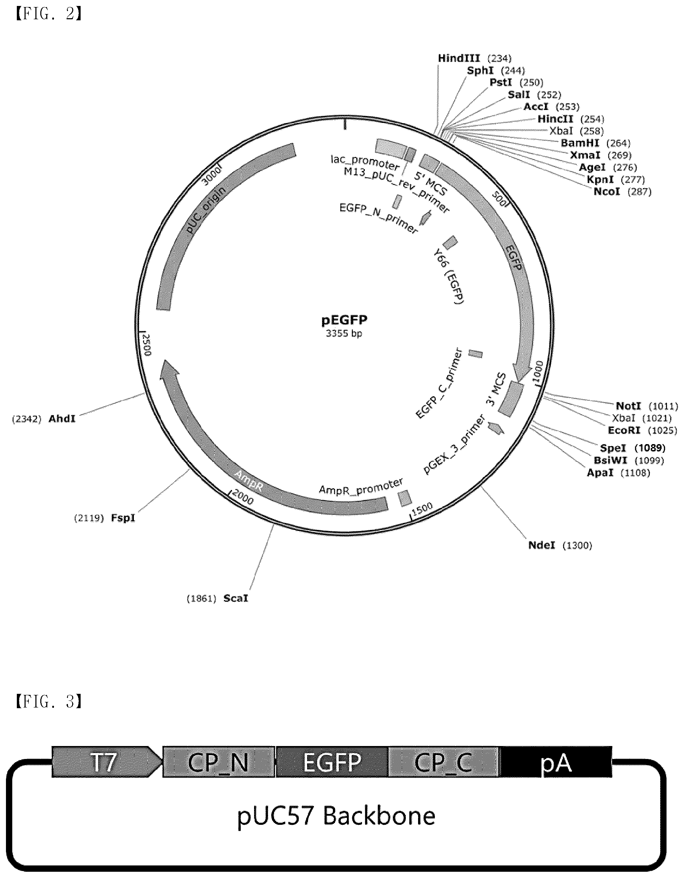

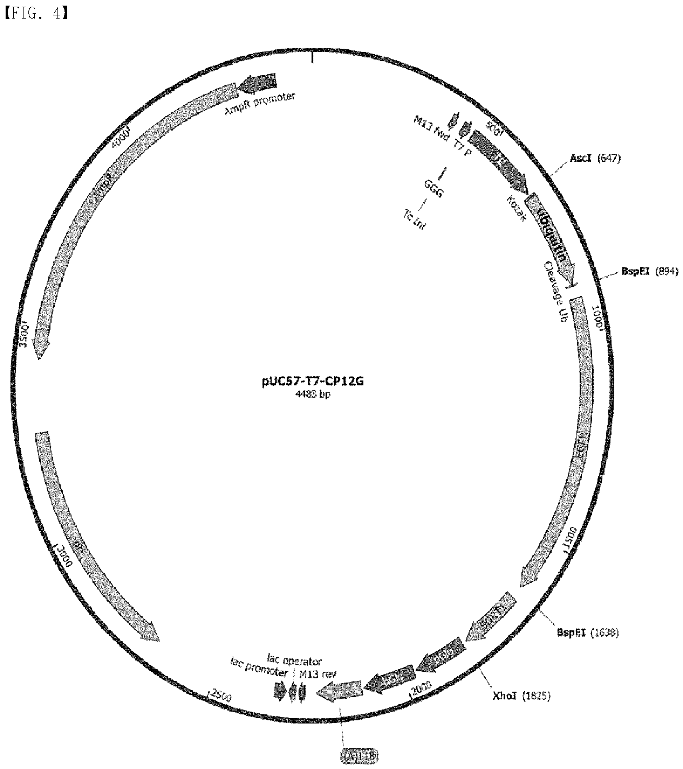

The vector map of the pEGFP vector was shown in FIG. 2, and the schematic diagram of the produced carrier protein vector was shown in FIG. 3, and the vector map of the exemplarily produced pUC57-T7-CP12G plasmid was shown in FIG. 4, and the amino acid and base sequence information of the carrier protein inserted into each of the N-terminus and C-terminus was shown in Tables 4 and 5 below.

**[Table 4]**

| Carrier protein type | Insertion location | Sequence (5' -> 3') | SEQ ID NO: |
|---|---|---|---|
| UbR | N-term | MQIFVKTLTGKTITLEVEPSDTIENVKAKIQDKEGIPPDQQRLIFAGKQLEDGRTLSD YNIQKESTLHLVLRLRGGR | 1 |
| Ub | C-term | | 2 |
| Lamp | N-term | MDDQRDL I SNNEQLPMLGRRPGAPESKCSR | 3 |
| | C-term | DDQRDLISNNEQLPMLGRRPGAPESKCSR | 4 |
| mODC | N-term | MLMKQIQSHGFPPEVEEQDDGTLPMSCAQESGMDRHPAACASARINVM | 5 |
| | C-term | LMKQIQSHGFPPEVEEQDDGTLPMSCAQESGMDRHPAACASARINVM | 6 |
| cmyc | N-term | MHEETPPTTSSDSEEEQEDEEEIDVVSVEK | 7 |
| | C-term | HEETPPTTSSDSEEEQEDEEEIDVVSVEK | 8 |
| SORT1 | N-term | MKKYVCGGRFLVHRYSVLQQHAEANGVDGVDALDTASHTNKSGYHDDSDEDLLE | 9 |
| | C-term | KKYVCGGRFLVHRYSVLQQHAEANGVDGVDALDTASHTNKSGYHDDSDEDLLE | 10 |
| TRXwt | N-term | MVKQIESKTAFQEALDAAGDKLVVVDFSATWCGPCKMIKPFFHSLSEKYSNVIFLEVD VDDCQDVASECEVKCMPTFQFFKKGQKVGEFSGANKEKLEATINELV | 11 |
| | C-term | VKQIESKTAFQEALDAAGDKLVVVDFSATWCGPCKMIKPFFHSLSEKYSNVIFLEVDV DDCQDVASECEVKCMPTFQFFKKGQKVGEFSGANKEKLEATINELV | 12 |
| PSBD | C-term | VIAMPSVRKYAREKGVDIRLVQGTGKGGRVLKEDIDAWLA | 13 |
| TRXwt-UbR | N-term | MVKQIESKTAFQEALDAAGDKLVVVDFSATWCGPCKMIKPFFHSLSEKYSNVIFLEVD VDDCQDVASECEVKCMPTFQFFKKGQKVGEFSGANKEKLEATINELVGGSGGGGSGGQ IFVKTLTGKTITLEVEPSDTIENVKAKIQDKEGIPPDQQRLIFAGKQLEDGRTLSDYN IQKESTLHLVLRLRGGR | 14 |
| TRXwt-Lamp | N-term | MVKQIESKTAFQEALDAAGDKLVVVDFSATWCGPCKMIKPFFHSLSEKYSNVIFLEVD VDDCQDVASECEVKCMPTFQFFKKGQKVGEFSGANKEKLEATINELVGGSGGGGSGGD DQRDLISNNEQLPMLGRRPGAPESKCSR | 15 |
| TRXwt-mODC | N-term | MVKQIESKTAFQEALDAAGDKLVVVDFSATWCGPCKMIKPFFHSLSEKYSNVIFLEVD VDDCQDVASECEVKCMPTFQFFKKGQKVGEFSGANKEKLEATINELVGGSGGGGSGGL MKQIQSHGFPPEVEEQDDGTLPMSCAQESGMDRHPAACASARINVM | 16 |
| TRXwt-cmyc | N-term | MVKQIESKTAFQEALDAAGDKLVVVDFSATWCGPCKMIKPFFHSLSEKYSNVIFLEVD VDDCQDVASECEVKCMPTFQFFKKGQKVGEFSGANKEKLEATINELVGGSGGGGSGGH EETPPTTSSDSEEEQEDEEEIDVVSVEK | 17 |
| TRXwt-SORT1 | N-term | MVKQIESKTAFQEALDAAGDKLVVVDFSATWCGPCKMIKPFFHSLSEKYSNVIFLEVD VDDCQDVASECEVKCMPTFQFFKKGQKVGEFSGANKEKLEATINELVGGSGGGGSGGK KYVCGGRFLVHRYSVLQQHAEANGVDGVDALDTASHTNKSGYHDDSDEDLLE | 18 |
| SORT1 w/ TM | C-term | SVPIILAIVGLMLVTVVAGVLIVKKYVCGGRFLVHRYSVLQQHAEANGVDGVDALDTA SHTNKSGYHDDSDEDLLE | 19 |
| Lamp w/ TM | N-term | MDDQRDLISNNEQLPMLGRRPGAPESKCSRGALYTGFSILVTLLLAGQATTAYFLY | 20 |
| TRXmt | N-term | MVKQIESKTAFQEALDAAGDKLVVVDFSATWSGPSKMIKPFFHSLSEKYSNVIFLEVD VDDSQDVASESEVKSMPTFQFFKKGQKVGEFSGANKEKLEATINELV. | 145 |

| | | | |
|---|---|---|---|
| (In Table 4 above, when the carrier protein is inserted into the N-term, methionine (M) having atg base sequence at the 5' end of each protein is added and inserted, and it corresponds to the GS linker sequence indicated by the underline above) | | | |

**[Table 5]**

| Carrier protein type | Insertion location | Sequence (5' -> 3') | SEQ ID NO: |
|---|---|---|---|
| UbR | N-term | atgcagatcttcgtgaaaaccctgaccggcaagaccatcacactggaagtggaacccag cgacaccatcgagaacgtgaaggccaagatccaggacaaagagggcatccctcctgacc agcagagactgatcttcgccggcaagcagctggaagatggcagaaccctgagcgactac aacatccagaaagagtctaccctgcacctggtgctgagactgagaggtggaaga | 21 |
| Ub | C-term | cagatcttcgtgaaaaccctgaccggcaagaccatcacactggaagtggaacccagcga caccatcgagaacgtgaaggccaagatccaggacaaagagggcatccctcctgaccagc agagactgatcttcgccggcaagcagctggaagatggcagaaccctgagcgactacaac atccagaaagagtctaccctgcacctggtgctgagactgagaggtgga | 22 |
| Lamp | N-term | atggacgaccagagggacctgatcagcaacaacgagcagctgcccatgctcggcagaag gcctggcgctcctgagagcaaatgtagcaga | 23 |
| | C-term | gacgaccagagggacctgatcagcaacaacgagcagctgcccatgctcggcagaaggcc tggcgctcctgagagcaaatgtagcaga | 24 |
| mODC | N-term | atgctgatgaagcagatccagagccacggcttcccacctgaggtggaagaacaggacga tggcaccctgcctatgagctgtgcccaagagtccggcatggatagacatcctgccgcct gtgccagcgccagaatcaatgttatg | 25 |
| | C-term | ctgatgaagcagatccagagccacggcttcccacctgaggtggaagaacaggacgatgg caccctgcctatgagctgtgcccaagagtccggcatggatagacatcctgccgcctgtg ccagcgccagaatcaatgttatg | 26 |
| cmyc | N-term | atgcacgaggaaacccctccaaccacaagcagcgacagcgaggaagaacaagaggacga ggaagagatcgacgtcgtcagcgtggaaaag | 27 |
| | C-term | cacgaggaaacccct ccaaccacaagcagcgacagcgaggaagaacaagaggacgagga agagat cgacgt cgt cagcgt ggaaaag | 28 |
| SORT1 | N-term | atgaagaaatacgtgtgcggcggcagattcctggtgcacagatatagcgtcctgcagca gcacgccgaggccaatggtgttgatggtgtcgatgccctggataccgccagccacacaa acaagagcggctaccacgacgacagcgacgaggatctgcttgag | 29 |
| | C-term | aagaaatacgtgtgcggcggcagattcctggtgcacagatatagcgtcctgcagcagca cgccgaggccaatggtgttgatggtgtcgatgccctggataccgccagccacacaaaca agagcggctaccacgacgacagcgacgaggatctgcttgag | 30 |
| TRXwt | N-term | atggtcaagcagatcgagagcaagaccgccttccaagaggcccttgatgccgctggcga taagctggtggtggtggatttttccgccacttggtgcggcccctgcaagatgatcaagc cattcttccacagcctgagcgagaagtacagcaacgtgatcttcctggaagtggacgtg gacgactgccaggatgtggcctctgagtgcgaagtgaagtgcatgcccaccttccagtt cttcaagaaaggccagaaagtgggcgagttcagcggcgccaacaaagagaagctggaag ccaccatcaacgaactagtt | 31 |
| | C-term | gtcaagcagatcgagagcaagaccgccttccaagaggcccttgatgccgctggcgataa gctggtggtggtggatttttccgccacttggtgcggcccctgcaagatgatcaagccat tcttccacagcctgagcgagaagtacagcaacgtgatcttcctggaagtggacgtggac gactgccaggatgtggcctctgagtgcgaagtgaagtgcatgcccaccttccagttctt caagaaaggccagaaagtgggcgagttcagcggcgccaacaaagagaagctggaagcca ccatcaacgaactagtt | 32 |
| PSBD | C-term | gtgatcgccatgcctagcgtgcggaagtacgccagagaaaaaggcgtggacatccggct ggtgcaaggcactggaaaaggcggcagagtgctgaaagaggacatcgacgcttggctgg ct | 33 |
| TRXwt-UbR | N-term | atggtcaagcagatcgagagcaagaccgccttccaagaggcccttgatgccgctggcga taagctggtggtggtggatttttccgccacttggtgcggcccctgcaagatgatcaagc cattcttccacagcctgagcgagaagtacagcaacgtgatcttcctggaagtggacgtg gacgactgccaggatgtggcctctgagtgcgaagtgaagtgcatgcccaccttccagtt cttcaagaaaggccagaaagtgggcgagttcagcggcgccaacaaagagaagctggaag ccaccatcaacgaactagttggcggatctggcggcggaggaagcggaggacagatcttc gtgaaaaccctgaccggcaagaccatcacactggaagtggaacccagcgacaccatcga gaacgtgaaggccaagatccaggacaaagagggcatccctcctgaccagcagagactga tcttcgccggcaagcagctggaagatggcagaaccctgagcgactacaacatccagaaa gagtctaccctgcacctggtgctgagactgagaggtggaaga | 34 |
| TRXwt-Lamp | N-term | atggtcaagcagatcgagagcaagaccgccttccaagaggcccttgatgccgctggcga taagctggtggtggtggatttttccgccacttggtgcggcccctgcaagatgatcaagc cattcttccacagcctgagcgagaagtacagcaacgtgatcttcctggaagtggacgtg gacgactgccaggatgtggcctctgagtgcgaagtgaagtgcatgcccaccttccagtt cttcaagaaaggccagaaagtgggcgagttcagcggcgccaacaaagagaagctggaag ccaccatcaacgaactagttggcggatctggcggcggaggaagcggaggagacgaccag agggacctgatcagcaacaacgagcagctgcccatgctcggcagaaggcctggcgctcc tgagagcaaatgtagcaga | 35 |
| TRXwt -mODC | N-term | atggtcaagcagatcgagagcaagaccgccttccaagaggcccttgatgccgctggcga taagctggtggtggtggatttttccgccacttggtgcggcccctgcaagatgatcaagc cattcttccacagcctgagcgagaagtacagcaacgtgatcttcctggaagtggacgtg gacgactgccaggatgtggcctctgagtgcgaagtgaagtgcatgcccaccttccagtt cttcaagaaaggccagaaagtgggcgagttcagcggcgccaacaaagagaagctggaag ccaccatcaacgaactagttggcggatctggcggcggaggaagcggaggactgatgaag cagatccagagccacggcttcccacctgaggtggaagaacaggacgatggcaccctgcc tatgagctgtgcccaagagtccggcatggatagacatcctgccgcctgtgccagcgcca gaatcaatgttatg | 36 |
| TRXwt-cmyc | N-term | atggtcaagcagatcgagagcaagaccgccttccaagaggcccttgatgccgctggcga taagctggtggtggtggatttttccgccacttggtgcggcccctgcaagatgatcaagc cattcttccacagcctgagcgagaagtacagcaacgtgatcttcctggaagtggacgtg gacgactgccaggatgtggcctctgagtgcgaagtgaagtgcatgcccaccttccagtt cttcaagaaaggccagaaagtgggcgagttcagcggcgccaacaaagagaagctggaag ccaccatcaacgaactagttggcggatctggcggcggaggaagcggaggacacgaggaa acccctccaaccacaagcagcgacagcgaggaagaacaagaggacgaggaagagatcga cgtcgtcagcgtggaaaag | 37 |
| TRXwt -SORT1 | N-term | atggtgaagcagatcgagagcaagaccgccttccaagaggcccttgatgccgctggcga taagctggtggtggtggatttttccgccacttggtgcggcccctgcaagatgatcaagc cattcttccacagcctgagcgagaagtacagcaacgtgatcttcctggaagtggacgtg gacgactgccaggatgtggcctctgagtgcgaagtgaagtgcatgcccaccttccagtt cttcaagaaaggccagaaagtgggcgagttcagcggcgccaacaaagagaagctggaag ccaccatcaacgaactagttggcggatctggcggcggaggaagcggaggaaagaaatac gtgtgcggcggcagattcctggtgcacagatatagcgtcctgcagcagcacgccgaggc caatggtgttgatggtgtcgatgccctggataccgccagccacacaaacaagagcggct accacgacgacagcgacgaggatctgcttgag | 38 |
| SORT1 w/ TM | C-term | agcgtgccaatcatcctggccatcgtgggcctgatgctggttacagtggttgccggcgt gctgatcgttaagaaatacgtgtgcggcggcagattcctggtgcacagatatagcgtcc tgcagcagcacgccgaggccaatggtgttgatggtgtcgatgccctggataccgccagc cacacaaacaagagcggctaccacgacgacagcgacgaggatctgcttgag | 39 |
| Lamp w/ TM | N-term | atggacgaccagagggacctgatcagcaacaacgagcagctgcccatgctcggcagaag gcctggcgctcctgagagcaaatgtagcagaggcgctctgtacaccggctttagcatcc tggtcacactgttgctggccggacaggctaccacagcctactttctgtac | 40 |
| TRXmt | N-term | atggtgaagcagatcgagagcaagaccgccttccaagaggcccttgatgccgctggcga taagctggtggtggtggatttttccgccacttggagcggccccagcaagatgatcaagc cattcttccacagcctgagcgagaagtacagcaacgtgatcttcctggaagtggacgtg gacgacagccaggatgtggcctctgagagcgaagtgaagtccatgcctaccttccagtt cttcaagaagggccagaaagtcggcgagttcagcggcgccaacaaagagaagctggaag ccaccatcaacgagctggtg | 146 |

| | | | |
|---|---|---|---|
| (In Table 5 above, when the carrier protein is inserted into the N-term, atg base sequence is added and inserted into the 5' end of each protein, and it corresponds to the GS linker sequence indicated by the underline above) | | | |

The mRNA of the following structural formula 1 was produced by in vitro transcription using mMESSAGE mMACHINE^{™} T7 ULTRA Transcription Kit (ThermoFisher, AM1345) for the produced carrier protein vector, and sequence information corresponding to 5'UTR and 3'UTR below was shown in Table 6 below.

[Structural formula 1] 5'UTR (TE) - (Carrier protein I) - GGSGGGGSGG - EGFP - GGSGGGGSGG - (Carrier protein II) - 3'UTR (2X HBB)

(In the structural formula 1, GGSGGGGSGG means a linker sequence, and Carrier protein I (CP #1) corresponds to a carrier protein inserted into the N-terminus of the produced vector, and Carrier protein II (CP #2) corresponds to a carrier protein inserted into the C-terminus, respectively, and the 5'UTR (TE) has the sequence of SEQ ID NO: 41 of Table 6 below, and the 3'UTR (2X HBB) has the sequence of SEQ ID NO: 42 of Table 6 below)

**[Table 6]**

| Classification | Sequence (5' -> 3') | SEQ ID NO: |
|---|---|---|
| 5UTR_TE | ccggcgggtttctgacatccggcgggtttctgacatccggcgggtttctgacatccggcgggtttc tgacatccggcgggtttctgacatccggcgggtttctgacatccggcgggtttctgacatccggcg ggtttctgacatccggcgggtttctgacatccggcgggtttctgacattcacaaccaggcctccac aacc | 41 |
| 3UTR_2X_HBB | agctcgctttcttgctgtccaatttctattaaaggttcctttgttccctaagtccaactactaaac tgggggatattatgaagggccttgagcatctggattctgcctaataaaaaacatttattttcattg ctgcggatctagctcgctttcttgctgtccaatttctattaaaggttcctttgttccctaagtcca actactaaactgggggatattatgaagggccttgagcatctggattctgcctaataaaaaacattt attttcattgctgc | 42 |

### Example 1-2. Carrier protein primary screening

When the expression pattern is changed by a proteasome inhibitor, BTZ (bortezomib), a CD8 antigen can be appropriately delivered to the processing/presentation pathway, and when the expression pattern is changed by a lysosomal inhibitor, CHQ (Chloroquine), a CD4 antigen can be appropriately delivered to the processing/presentation pathway, and thus, by treatment of BTZ and CHQ, whether processing/presentation of the CD8 and CD4 antigens is performed can be determined, so BTZ and CHQ were treated.

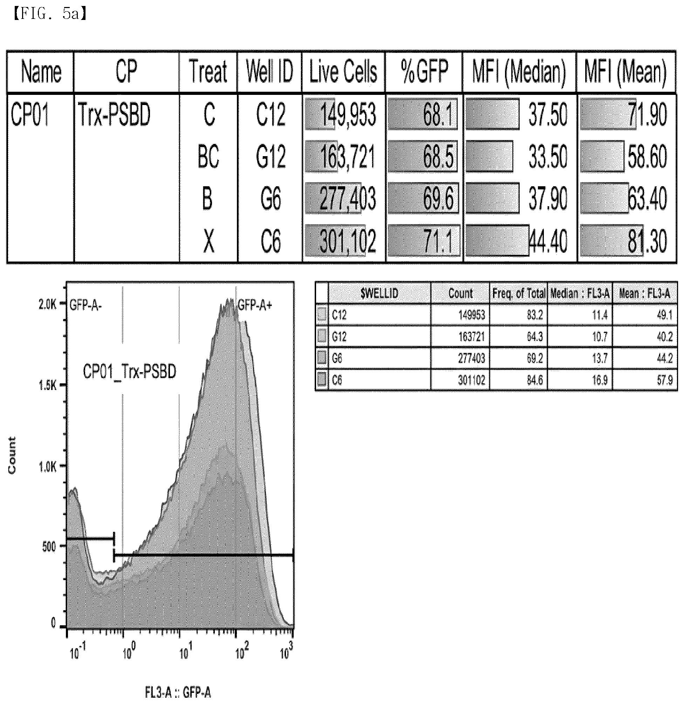

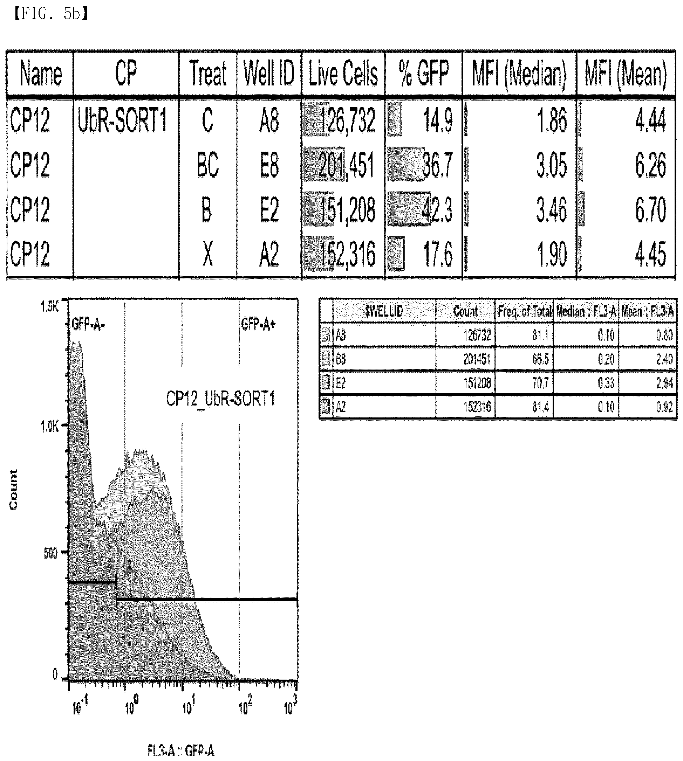

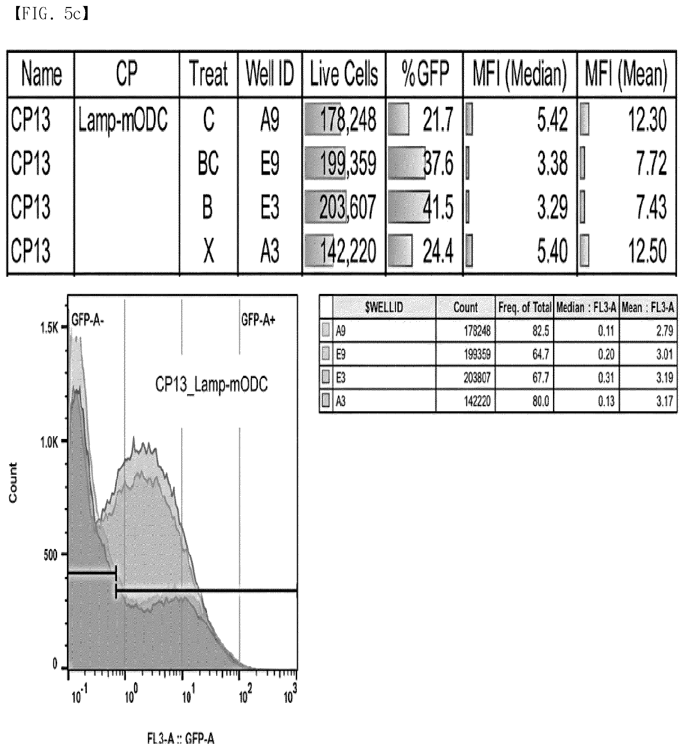

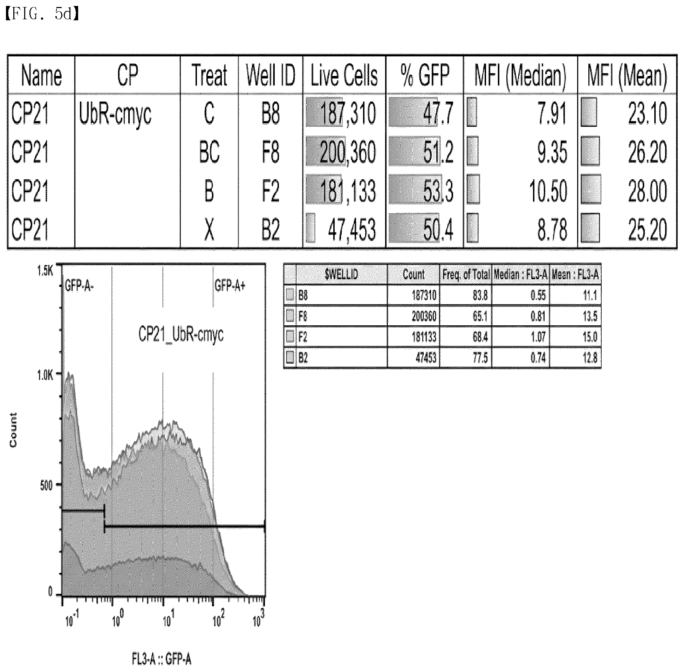

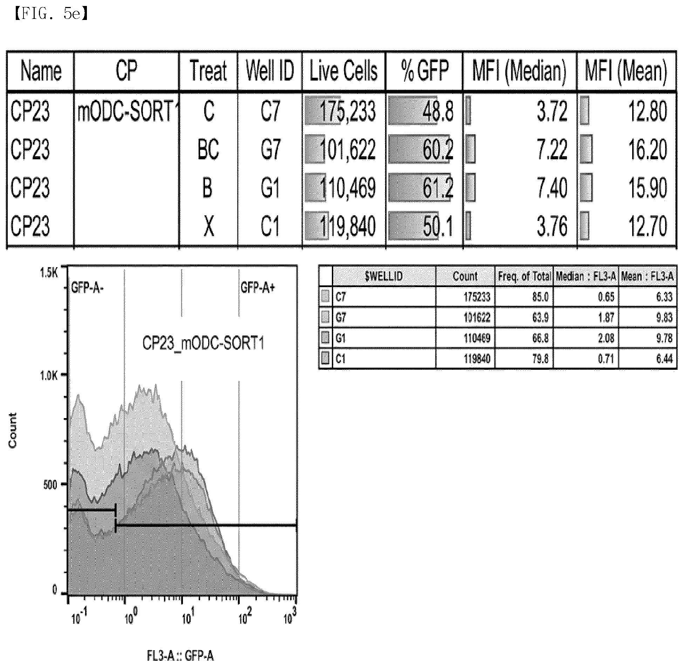

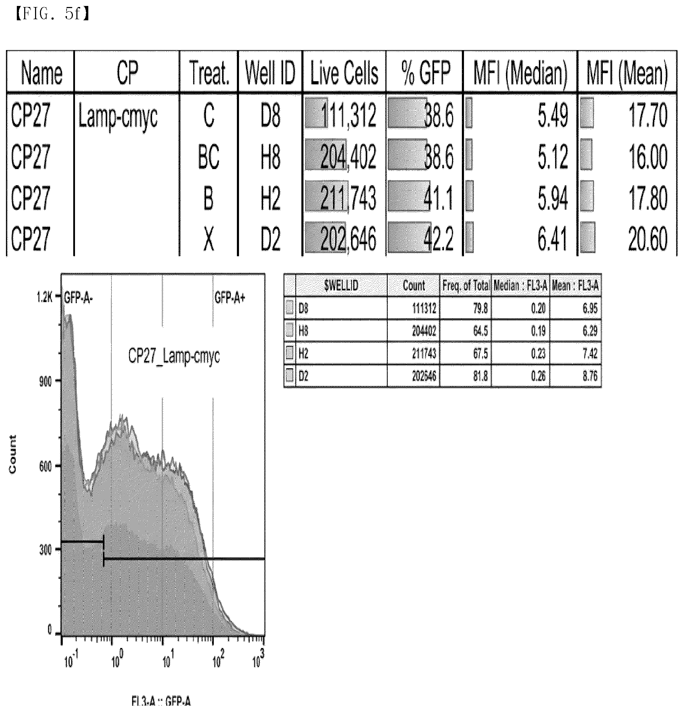

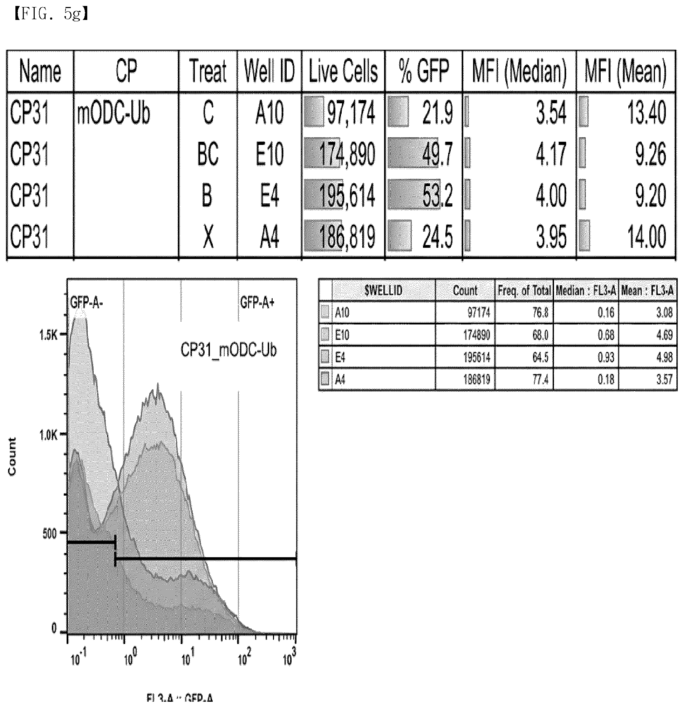

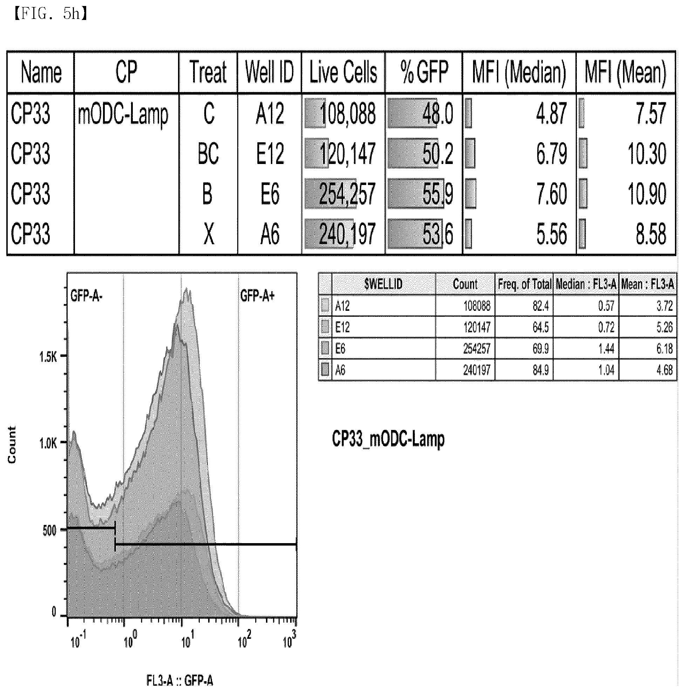

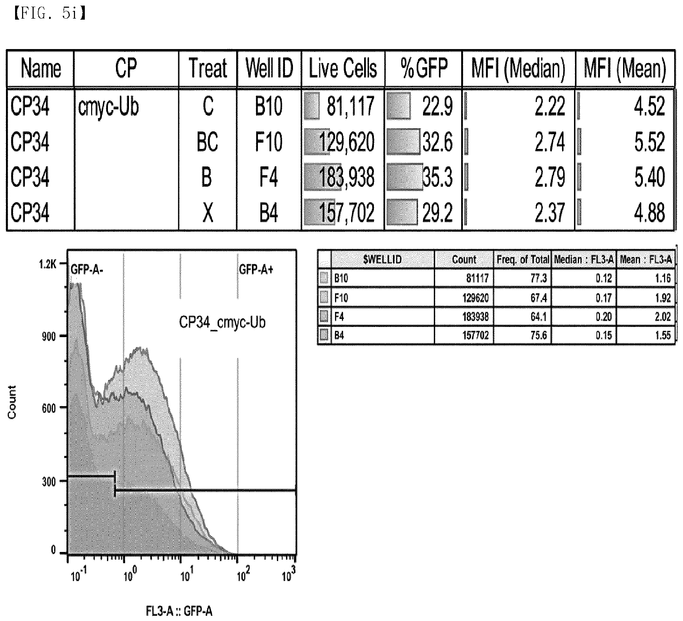

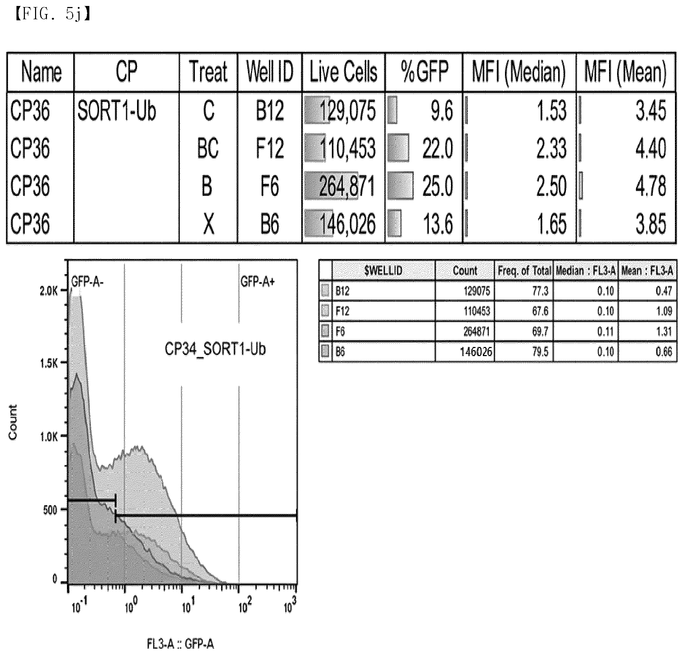

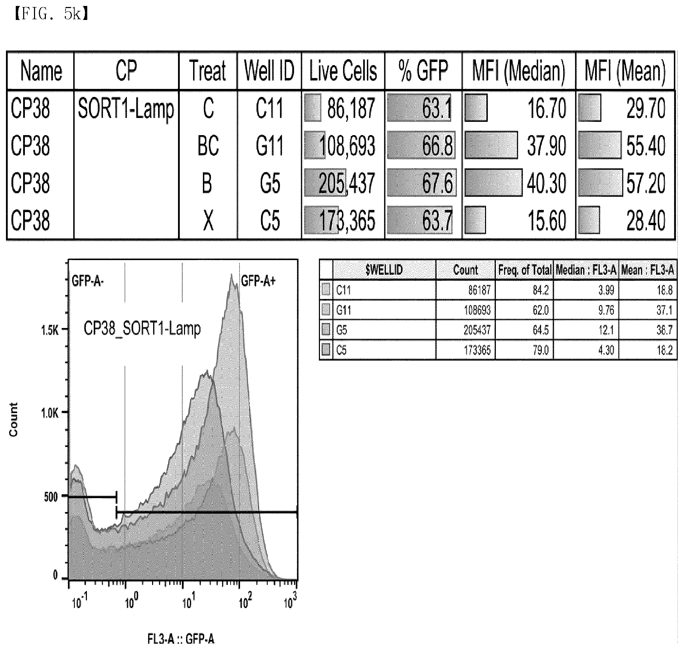

Specifically, each of the produced mRNA was transfected into the K562 Cell line (ATCC, CCL-243) using 4D-nucleofector system (Lonza). For the transfected cells, to confirm the response to each inhibitor using the proteasome inhibitor, Bortezomib (BTZ), and the lysosomal inhibitor, Chloroquine (CHQ), BTZ or CHQ was treated to the cells, and after 16 hours, the reactivity was confirmed using a fluorescence-activated cell sorter (FACS), to confirm whether degradation by proteasome or lysosome was inhibited, and thereby, the possibility of delivery to HLA Class I and Class II pathways was confirmed, and the result of confirming the possibility of delivery of the above combination was shown in FIGs. 5a to 5k.

Specifically, in FIGs. 5a to 5k above, C refers to CHQ treatment, and B refers to BTZ treatment, and BC refers to treatment of both BTZ and CHQ, and X refers to that nothing is treated, and when GFP expression increases by C compared to X above, it means that the possibility to go to the CD4 antigen is high, and when the expression increases by B compared to the X, it means that that to the CD8 antigen is high, and when the expression increases by BC compared to the X, that to both the CD4 and CD8 antigens well is high.

As a result of confirming the possibility of delivery, the CP01 (TRXmt-PSBD) combination in which the mutated TRX was inserted was confirmed that EGFP was expressed well without BTZ and CHQ effects, and the CP12, CP13, CP23, CP31, CP33, CP34, CP36 and CP38 combination of Table 1 above were confirmed to be affected by BTZ, and it was confirmed that there was no combination affected by CHQ, and thus, it was confirmed that the CD4 and CD8 antigens were delivered to the processing/presentation pathway by the CP01 combination, and the CD8 antigen was delivered to the processing/presentation pathway by the CP12, CP13, CP23, CP31, CP33, CP34, CP36 and CP38 combinations. In addition, the CP21 (UbR-cmyc), and CP27 (Lamp-cmyc) combinations could not distinguish the effects of BTZ and CHQ.

### Example 1-3. Carrier protein secondary screening

Since it was confirmed that the CP01 combination allowed both the CD4 and CD8 antigens to be delivered to the processing/presentation pathway in Example 1-2 above, a design to add TRX of the CP01 combination to the CP12, CP13, CP23, CP31, CP33, CP34, CP36 and CP38 combinations confirmed to allow the CD8 antigen to be delivered to the processing/presentation pathway in Example 1-2 above was done.

Specifically, using each plasmid of pUC57-T7-CP12G, pUC57-T7-CP13G, pUC57-T7-CP23G, pUC57-T7-CP31G, pUC57-T7-CP33G, pUC57-T7-CP34G, pUC57-T7-CP36G and pUC57-T7-38G produced in Example 1-1 above as templates, and oligonucleotides oCCP12G-F1 and oCCPxxG-R1 of Table 7 below as primers, PCR was performed, and each product was purified.

**[Table 7]**

| Classification | Sequence (5' -> 3') | SEQ ID NO: |
|---|---|---|
| oCCP12G-F1 | ggaggaagcggaggacagatcttcgtgaaa | 43 |
| oCCPxxG-R1 | gcaagaaagcgagctctcgagttta | 44 |

After purification, with the TRX PCR product produced during the process of producing plasmids in Example 1-1 above, a plasmid in which TRX was added to the N-terminus finally by insertion by EZ-fusion method to the backbone part purified by treating AscI (Enzynomics; R068M) and XhoI (Thermo Scientific^{™} FastDigest; #FD0694) restriction enzymes to each plasmid of pUC57-T7-CP12G, pUC57-T7-CP13G, pUC57-T7-CP23G, pUC57-T7-CP31G, pUC57-T7-CP33G, pUC57-T7-CP34G, pUC57-T7-CP36G and pUC57-T7-38G produced in Example 1-1 was produced, and the produced plasmid was named by adding 1 in front of the number of the original plasmid (for example, plasmid in which TRX is added to pUC57-T7-CP12G: pUC57-T7-CP112G).

In addition, for the CP12, CP13, CP21, CP31 and CP33 combinations, the carrier protein at the N-terminus was changed to TRX (CP41 to CP45).

Specifically, using the pUC57-T7-CP21G, pUC57-T7-CP33G, pUC57-T7-CP-13G, pUC57-T7-CP12G and pUC57-T7-CP31G plasmids of Example 1-1 as templates, and oligonucleotides oGS-EGFP-F and oCCPxxG-R1 of Table 8 below as primers, PCR was performed, and each product was purified, and fragments in which EGFP and the carrier protein at the C-terminus were connected were obtained.

**[Table 8]**

| Classification | Sequence (5' -> 3') | SEQ ID NO: |
|---|---|---|
| oGS-EGFP-F | ggcggatctggcggcggaggaagcggaggagtgagcaagggcgag | 45 |
| oCCPxxG-R1 | gcaagaaagcgagctctcgagttta | 46 |

After that, with the TRX PCR product produced in Example 1-1 above, plasmids in which the carrier protein at the N-terminus of each plasmid was TRX was finally produced by insertion by EZ-fusion method into the backbone part purified by treating AscI (Enzynomics; R068M) and XhoI (Thermo Scientific^{™} FastDigest; #FD0694) restriction enzymes to each plasmid of pUC57-T7-CP21G, pUC57-T7-CP33G, pUC57-T7-CP-13G, pUC57-T7-CP12G and pUC57-T7-CP31G produced in Example 1-1 (pUC57-T7-41G, pUC57-T7-CP42G, pUC57-T7-CP43G, pUC57-T7-44G and pUC57-T7-CP45G).

In addition, for the CP34, CP27, CP23, CP36 and CP21 combinations, the carrier protein at the C-terminus was changed to TRX (CP46 to CP50).

Specifically, using the pUC57-T7-CP34G, pUC57-T7-CP27G, pUC57-T7-CP23G, pUC57-T7-CP36G and pUC57-T7-CP21G plasmids of Example 1-1 as templates, and the oM13F and oGS-EGFP-R of Table 9 below as primers, PCR was performed, and each product was purified, and thereby, fragments in which EGFP and the carrier protein at the N-terminus were connected were obtained.

**[Table 9]**

| Classification | Sequence (5' -> 3') | SEQ ID NO: |
|---|---|---|
| oM13F | gtaaaacgacggccagt | 47 |
| oGS-EGFP-R | tcctccgcttcctccgccgccagatccgcccttgtacagctcgtc | 48 |

After that, using pUC57-T7-CP01G produced in Example 1-1 as a template, PCR was performed with oligonucleotides oCTRXwt-F1 and oCTRXwt-R1 as primers (Table 10) to obtain the TRX fragment for C-terminal insertion. This fragment, together with the fragments in which EGFP and the carrier protein at the N-terminus were connected, was then inserted by the EZ-fusion method into each of pUC57-T7-CP34G, pUC57-T7-CP27G, pUC57-T7-CP23G, pUC57-T7-CP36G, and pUC57-T7-CP21G, which had been treated with AscI (Enzynomics; R068M) and XhoI (Thermo Scientific^{™} FastDigest; #FD0694) restriction enzymes, thereby producing the final plasmids pUC57-T7-46G, pUC57-T7-CP47G, pUC57-T7-CP48G, pUC57-T7-49G, and pUC57-T7-CP50G.

**[Table 10]**

| Classification | Sequence (5' -> 3') | SEQ ID NO: |
|---|---|---|
| oCTRXwt-F1 | ggcggatctggcggcggaggaagcggaggagtcaagcagatcgag | 49 |
| oCTRXwt-R1 | gcaagaaagcgagctctcgagtttaaactagttcgttgat | 50 |

The carrier protein information inserted into each of the N-terminus and C-terminus of the designed vector was shown in Table 2 above, and the amino acid sequence and base sequence information of the carrier protein in which TRX among the carrier proteins was added was shown in Tables 4 and 5 above, respectively.

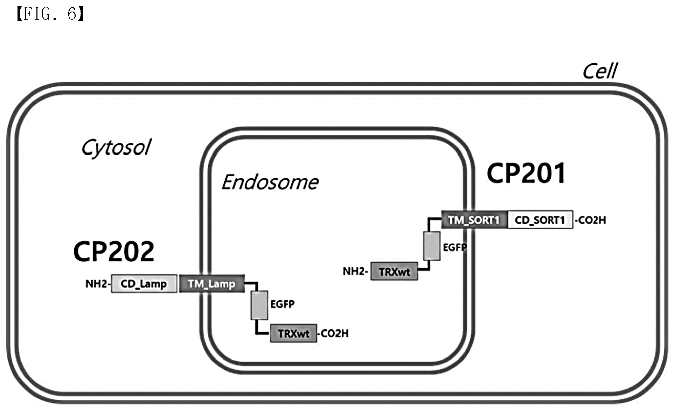

In addition, since SORT1 and Lamp among the carrier proteins have a transmembrane as a membrane protein, the transmembrane domain was added to the existing cytosolic domain of each protein (SORT1 w/ TM and Lamp w/ TM), and SORT1 w/ TM was made to be located at the C-term, and TRX was made to be located to the N-term, or Lamp w/ TM was made to be located at the N-term and TRX was made to be located at the C-term (CP201 and CP202), and the schematic diagram of intracellular expression was shown in FIG. 6.

Specifically, the SORT1 w/ TM was produced by performing PCR by the substantially same method as SORT1 using the oligonucleotides of oSORTwTM-1F, oSORTwTM-4R, oSORTwTM-2R and oSORTwTM-3F of Table 3 above, and the Lamp w/ TM was produced by performing PCR by the substantially same method as SORT1 using the oligonucleotides of oLAMPwTM-1F, oLAMPwTM-4R, oLAMPwTM-2R, and oLAMPwTM-3F of Table 3 above.

The carrier protein information in which the transmembrane domain of the produced vector was added was shown in Table 2 above, and the amino acid sequence and base sequence information of the carrier protein in which the transmembrane was added was shown in Tables 4 and 5 above, respectively.

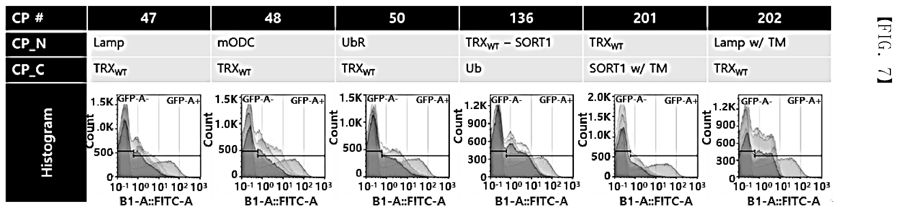

For the produced carrier protein vector, by the substantially same method as Example 1-1 above, the possibility to deliver to HLA Class I and Class II pathway after producing mRNA was confirmed, and the result was shown in FIG. 7.

As a result of confirming the possibility of delivery, it was confirmed that the existing response was strengthened in the CP136 combination in which TRX was added, and it was confirmed that a response to CHQ was shown, when the transmembrane was present in SORT1 and Lamp in the CP201 and CP202 combinations in which the transmembrane was added, and thus, it was confirmed that the overall response was strengthened.

### Example 2. Construction of vector in which carrier protein is inserted and epitope is comprised

### Example 2-1. Carrier protein vector construction

In order to confirm the effect of the CP12, CP13, CP23, CP31, CP33, CP34, CP36, CP38, CP47, CP48, CP50, CP136, CP201 and CP202 combinations of which possibility to deliver to the HLA Class I and Class II pathway was confirmed in Example 1 above, on the expression efficiency of an epitope, a new type of vector comprising the carrier protein combinations was produced.

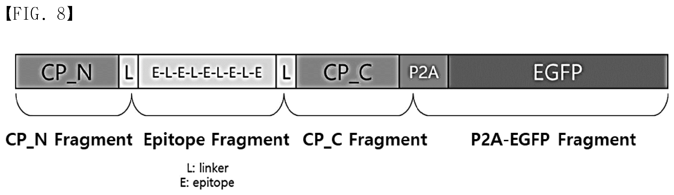

Specifically, in the above combinations, a vector was constructed in which five epitopes are linked as a linker between a carrier protein (CP_N; carrier protein 1) inserted at the N-terminus and a carrier protein (CP_C; carrier protein 2) inserted at the C-terminus, and EGFP is linked to the C-terminus of carrier protein 2 with a P2A sequence, and the schematic diagram of the constructed vector was shown in Fig. 8. In the vector of Example 1, EGFP was located between the two carrier proteins, and in the produced vector, as an actual application example, an epitope was located and EGFP for measuring the expression level was connected to the C-terminus.

For constructing the vector, an insert corresponding to a gene for cloning was produced by dividing into 4 fragments as in FIG. 8. The first fragment (CP_N fragment; CP#1) comprises a part of the first linker from the carrier protein 1, and the second fragment (epitope fragment; Epitope_2) comprises a part of the first linker and epitope and linker, and a part of the sixth linker, the third fragment (CP_C fragment; CP#3) comprises a part of the sixth linker, the carrier protein 2, and a part of EGFP sequence, and the fourth fragment (P2A-EGFP fragment; GFP_4) comprises a part of the P2A sequence, and EGFP sequence. The second fragment and the fourth fragment are used in common regardless of the carrier protein combination.

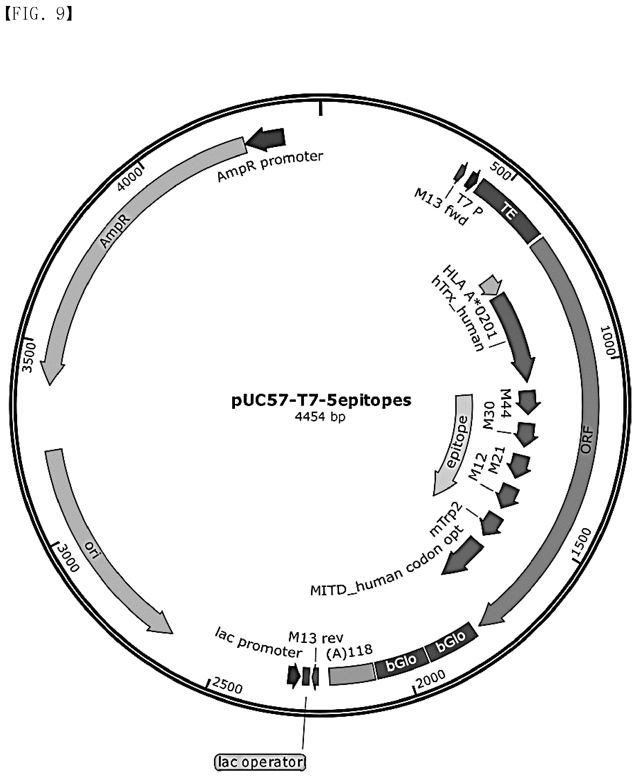

Using the primers of Table 12 capable of amplifying the carrier proteins listed below in the combinations of Table 11 according to the combinations of CP12, CP13, CP23, CP31, CP33, CP34, CP36, CP38, CP47, CP48, CP50, CP136, CP201, and CP202 of Table 2 above, PCR was performed on each vector manufactured in Example 1 to amplify the first and third fragments. Using the primers capable of amplifying the epitope (Epitope_2) and P2A-EGFP (GFP_4) of Table 12 below, PCR was performed on the pUC57-T7-5epitopes plasmid (Fig. 9) to amplify the second fragment, and the fourth fragment was also amplified using the pUC57-T7-CP12G plasmid of Example 1-1 as a template. Additionally, to prepare a plasmid with TRX inserted at the N-terminus and no carrier protein inserted at the C-terminus, PCR was performed using the primers in Table 12 below in the combinations in Table 11 (TRX_WT).

**[Table 11]**

| Combination name | Primer type used during CP #1 cloning | Primer type used during CP #2 cloning |
|---|---|---|
| hTrx_WT | TrxWT_1_F / TrxWT_1_R | - |
| CP12 | Ubi_1_F / Ubi_1_R | SORT1_3_F / SORT1_3_R |
| CP13 | Lamp_1_F / Lamp_1_R | mODC_3_F / mODC_3_R |
| CP23 | mODC_1_F / mODC_1_R | SORT1_3_F / SORT1_3_R |
| CP31 | mODC_1_F / mODC_1_R | Ubi_3_F / Ubi_3_R |
| CP33 | mODC_1_F / mODC_1_R | Lamp_3_F / Lamp_3_R |
| CP34 | cMyc_1_F / cMyc_1_R | Ubi_3_F / Ubi_3_R |
| CP36 | SORT1_1_F / SORTI_1_R | Ubi_3_F / Ubi_3_R |
| CP38 | SORT1_1_F / SORT1_1_R | Lamp_3_F / Lamp_3_R |
| CP47 | Lamp_1_F / Lamp_1_R | TrxWT_3_F / Tr x WT_3_R |
| CP48 | mODC_1_F / mODC_1_R | TrxWT_3_F / Tr x WT_3_R |
| CP50 | Ubi_1_F / Ubi_1_R | TrxWT_3_F / Tr x WT_3_R |
| CP136 | TrxWT_1_F / TrxWT_SORT1_1_R | Ubi_3_F / Ubi_3_R |
| CP201 | TrxWT_1_F / TrxWT_1_R | SORT1_TM_3_F / SORT1_3_R |
| CP202 | Lamp_1_F / Lamp_TM_1_R | TrxWT_3_F / TrxWT_3_R |

**[Table 12]**

| Primer name | Sequence (5' -> 3') | SEQ ID NO: |
|---|---|---|
| Epitope_2_F | GGCTACCGTCTCAAGGAAGCGGAGGTGAGTTCAAGCACATCAAGGCC | 114 |
| Epitope_2_R | GGCTACCGTCTCTACTGCCGCCATCTCTGACGCTGTAGTAGTG | 115 |
| GFP_4_F | GGCTACCGTCTCACTACTAACTTCAGCCTGCTGAAGCAGGCTGGCGACGTGGAGGAGAACCCTGGAC CTGTGAGCAAGGGCGAGGA | 116 |
| GFP_4_R | GGCTACCGTCTCTCGAGTTTACTTGTACAGCTCGTCCATG | 117 |
| cMyc_1_F | GGCTACCGTCTCACCATGCACGAGGAAACCCC | 118 |
| cMyc_1_R | GGCTACCGTCTCATCCTCCGCCGCCAGATCCGCCCTTTTCCACGCTGACGAC | 119 |
| Lamp_1_F | GGCTACCGTCTCACCATGGACGACCAGAGGGAC | 120 |
| Lamp_1_R | GGCTACCGTCTCATCCTCCGCCGCCAGATCCGCCTCTGCTACATTTGCTCTCAGGAG | 121 |
| Lamp_3_F | GGCTACCGTCTCTCAGTGGCGGAGGCGGCTCAGGCGGAGACGACCAGAGGGACCT | 122 |
| Lamp_3_R | GGCTACCGTCTCTGTAGCTCCGCTTCCAGCGTAATCTGGAACATCGTATGGGTATCCGGAGCCGCCT CTGCTACATTTGCTCTCAGGAG | 123 |
| Lamp_TM_1_R | GGCTACCGTCTCATCCTCCGCCGCCAGATCCGCCGTACAGAAAGTAGGCTGTGGT | 124 |
| mODC_1_F | GGCTACCGTCTCACCATGCTGATGAAGCAGATC | 125 |
| mODC_1_R | GGCTACCGTCTCATCCTCCGCCGCCAGATCCGCCCATAACATTGATTCTGGCGCT | 126 |
| mODC_3_F | GGCTACCGTCTCTCAGTGGCGGAGGCGGCTCAGGCGGACTGATGAAGCAGATCCAGAGCC | 127 |
| mODC_3_R | GGCTACCGTCTCTGTAGCTCCGCTTCCAGCGTAATCTGGAACATCGTATGGGTATCCGGAGCCGCCC ATAACATTGATTCTGGCGCTG | 128 |
| SORT1_1_F | GGCTACCGTCTCACCATGAAGAAATACGTGTGCGGC | 129 |
| SORT1_1_R | GGCTACCGTCTCATCCTCCGCCGCCAGATCCGCCCTCAAGCAGATCCTCGTCG | 130 |
| SORT1_3_F | GGCTACCGTCTCTCAGTGGCGGAGGCGGCTCAGGCGGAAAGAAATACGTGTGCGGC | 131 |
| SORT1_3_R | GGCTACCGTCTCTGTAGCTCCGCTTCCAGCGTAATCTGGAACATCGTATGGGTATCCGGAGCCGCCC TCAAGCAGATCCTCGTCG | 132 |
| SORT1_TM_3_F | GGCTACCGTCTCTCAGTGGCGGAGGCGGCTCAGGCGGAAGCGTGCCAATCATCCTGG | 133 |
| TrxWT_1_F | GGCTACCGTCTCACCATGGTGAAGCAGATCGAGAG | 134 |
| Tr x WT_1_R | GGCTACCGTCTCATCCTCCGCCGCCAGATCCGCCAACTAGTTCGTTGATGGTGGC | 135 |
| Tr x WT_3_F | GGCTACCGTCTCTCAGTGGCGGAGGCGGCTCAGGCGGAGTCAAGCAGATCGAGAGCAA | 136 |
| Tr x WT_3_R | GGCTACCGTCTCTGTAGCTCCGCTTCCAGCGTAATCTGGAACATCGTATGGGTATCCGGAGCCGCCA ACTAGTTCGTTGATGGTGGC | 137 |
| TrxWT_SORT1_1_R | GGCTACCGTCTCATCCTCCGCCGCCAGATCCGCCCTCAAGCAGATCCTCGTCG | 138 |
| Ubi_1_F | GGCTACCGTCTCACCATGCAGATCTTCGTGAAAACCC | 139 |
| Ubi_1_R | GGCTACCGTCTCATCCTCCGCCGCCAGATCCGCCTCTTCCACCTCTCAGTCTCAG | 140 |
| Ubi_3_F | GGCTACCGTCTCTCAGTGGCGGAGGCGGCTCAGGCGGACAGATCTTCGTGAAAACCCTGA | 141 |
| Ubi_3_R | GGCTACCGTCTCTGTAGCTCCGCTTCCAGCGTAATCTGGAACATCGTATGGGTATCCGGAGCCGCCT CCACCTCTCAGTCTCAGC | 142 |

The produced three kinds or four kinds of fragments according to each combination comprise BsmBI sites at both ends, and thus, they are inserted into an empty vector having BsmBI restriction enzyme sites, pLRK33-empty, using BsmBI restriction enzyme and T4 DNA Ligase of NEBridge Golden Gate Assembly-BsmBIv2; NEB # E1602L kit, and 5 kinds of epitopes are comprised in the epitope fragments, and based on the CP_N fragment location, they are located in the order of M44, M30, M21, M12 and mTrp2, and each epitope is connected by the linker of GGSGGGGSGG sequence. The M44, M30, M21 and M12 epitopes correspond to B16-M44, B16-M30, B16-M21 and B16-M12 disclosed in the paper of https://www.ncbi.nlm.nih.gov/pmc/articles/PMC4838069.

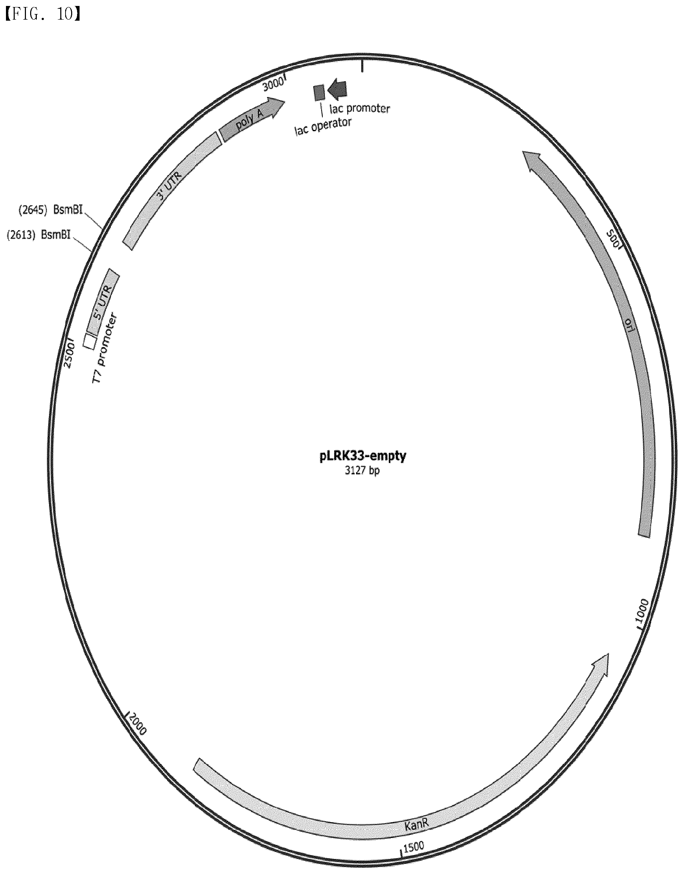

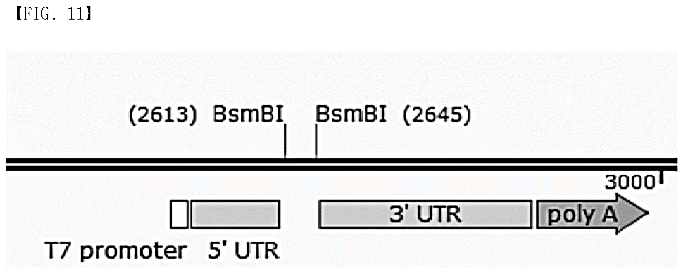

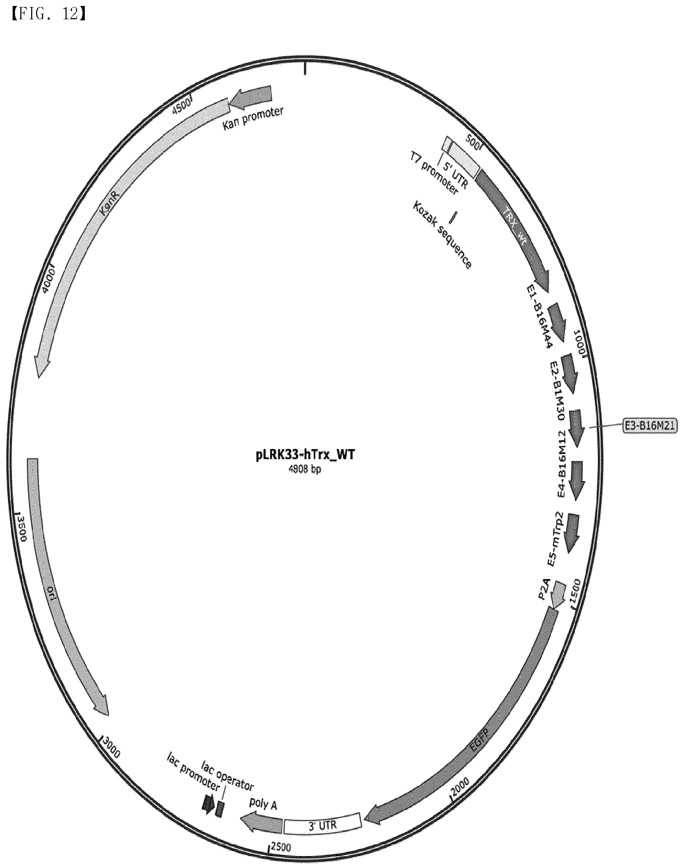

The vector map of the empty vector was shown in FIG. 10, and the schematic diagram showing the process of inserting each type of fragment into the empty vector was shown in FIG. 11, and the configuration information of the vector produced by being inserted into the empty vector was shown in Table 13 below, and the amino acid sequence and base sequence information of the inserted 5 kinds of epitope fragments and the amino acid sequence and base sequence information (5 kinds of epitopes) in a form in which the 5 kinds of epitope fragments were connected with a linker was shown in Table 14 below, and the amino acid sequence and base sequence information of the inserted P2A and EGFP was shown in Table 15 below. Exemplarily, the vector map of the produced plasmid pLRK33-hTrx_WT was shown in FIG. 12.

**[Table 13]**

| Plasmid type | CP_N fragment | Epitope fragment | CP_C fragment | P2A-EGFP fragment |
|---|---|---|---|---|
| pLRK33-hTrx_WT | Trxwt | | - | |
| pLRK33-CP12_m | UbR | | SORT1 | |
| pLRK33-CP13_m | Lamp | | mODC | |
| pLRK33-CP23_m | mODC | | SORT1 | |
| pLRK33-CP31_m | mODC | | Ub | |
| pLRK33-CP33_m | mODC | | Lamp | |
| pLRK33-CP34_m | cMyc | | Ub | |
| pLRK33-CP36_m | SORT1 | 5 kinds of epitopes | Ub | P2A-EGFP |
| pLRK33-CP38_m | SORT1 | | Lamp | |
| pLRK33-CP47_m | Lamp | | Trxwt | |
| pLRK33-CP48_m | mODC | | Trxwt | |
| pLRK33-CP50_m | UbR | | Trxwt | |
| pLRK33-CP136_m | Trxwt-SORT1 | | Ub | |
| pLRK33-CP201_m | Trxwt | | SORT1 w/TM | |
| pLRK33-CP202_m | Lamp w/TM | | Trxwt | |

**[Table 14]**

| Type | Sequence (5' -> 3') | SEQ ID NO: |
|---|---|---|
| M44_PRT | EFKHIKAFDRTFANNPGPMVVFATPGM | 51 |
| M44_GENE | gagttcaagcacatcaaggccttcgaccggaccttcgccaacaatcccggacctatggtggtgttcgccac acctggaatg | 52 |
| M30_PRT | PSKPSFQEFVDWENVSPELNSTDQPFL | 53 |
| M30_GENE | cctagcaagcctagcttccaagagttcgtggactgggagaacgtgtcccctgagctgaactctaccgacca gccatttctc | 54 |
| M21_PRT | SSPDEVALVEGVQSLGFTYLRLKDNYM | 55 |
| M21_GENE | tcttctccagatgaggtggcactggtggaaggcgtgcagtctctgggcttcacctacctgagactgaagga caactacatg | 56 |
| M12_PRT | TPPPEEAMPFEFNGPAQGDHSQPPLQV | 57 |
| M12_GENE | acacctccacctgaagaggccatgcctttcgagttcaacggacccgctcagggcgatcactctcagcctcc acttcaagtt | 58 |
| mTrp2_PRT | PNGTQPQIANCSVYDFFVWLHYYSVRD | 59 |
| mTrp2_GENE | cctaatggaacacagccccagatcgccaactgcagcgtgtacgacttcttcgtgtggctgcactactacag cgtcagagat | 60 |
| 5 Epitopes_PRT | GGSGGGGSGGEFKHIKAFDRTFANNPGPMVVFATPGMGGSGGGGSGGPSKPSFQEFVDWENVSPELNSTDQ PFLGGSGGGGSGGSSPDEVALVEGVQSLGFTYLRLKDNYMGGSGGGGSGGTPPPEEAMPFEFNGPAQGDHS QPPLQVGGSGGGGSGGPNGTQPQIANCSVYDFFVWLHYYSVRDGGSGGGGSGG | 61 |
| 5 Epitopes_GENE | ggcggatctggcggcggaggaagcggaggtgagttcaagcacatcaaggccttcgaccggaccttcgccaa caatcccggacctatggtggtgttcgccacacctggaatgggtggaagcggaggaggcggctctggaggcc ctagcaagcctagcttccaagagttcgtggactgggagaacgtgtcccctgagctgaactctaccgaccag ccatttctcggcggatcaggaggcgggggctccggagggtcttctccagatgaggtggcactggtggaagg cgtgcagtctctgggcttcacctacctgagactgaaggacaactacatgggaggatccggcggcggcggaa gcggggggacacctccacctgaagaggccatgcctttcgagttcaacggacccgctcagggcgatcactct cagcctccacttcaagttggcggatctggcggcggcggatcaggtggccctaatggaacacagccccagat cgccaactgcagcgtgtacgacttcttcgtgtggctgcactactacagcgtcagagatggcggcagtggcg gaggcggctcaggcgga | 62 |

**[Table 15]**

| Type | Sequence (5' -> 3') | SEQ ID NO: |
|---|---|---|
| P2A_PRT | ATNFSLLKQAGDVEENPGP | 63 |
| P2A_GENE | gctactaacttcagcctgctgaagcaggctggcgacgtggaggagaaccctggacct | 64 |
| EGFP_PRT | VSKGEELFTGVVPILVELDGDVNGHKFSVSGEGEGDATYGKLTLKFICTTGKLPVPWPTLVTTLTYGVQCFSR YPDHMKQHDFFKSAMPEGYVQERTIFFKDDGNYKTRAEVKFEGDTLVNRIELKGIDFKEDGNILGHKLEYNYN SHNVYIMADKQKNGIKVNFKIRHNIEDGSVQLADHYQQNTPIGDGPVLLPDNHYLSTQSALSKDPNEKRDHMV LLEFVTAAGITLGMDELYK | 65 |
| EGFP_GENE | gtgagcaagggcgaggagctgttcaccggggtggtgcccatcctggtcgagctggacggcgacgtaaacggcc acaagttcagcgtgtccggcgagggcgagggcgatgccacctacggcaagctgaccctgaagttcatctgcac caccggcaagctgcccgtgccctggcccaccctcgtgaccaccctgacctacggcgtgcagtgcttcagccgc taccccgaccacatgaagcagcacgacttcttcaagtccgccatgcccgaaggctacgtccaggagcgcacca tcttcttcaaggacgacggcaactacaagacccgcgccgaggtgaagttcgagggcgacaccctggtgaaccg catcgagctgaagggcatcgacttcaaggaggacggcaacatcctggggcacaagctggagtacaactacaac agccacaacgtctatatcatggccgacaagcagaagaacggcatcaaggtgaacttcaagatccgccacaaca tcgaggacggcagcgtgcagctcgccgaccactaccagcagaacacccccatcggcgacggccccgtgctgct gcccgacaaccactacctgagcacccagtccgccctgagcaaagaccccaacgagaagcgcgatcacatggtc ctgctggagttcgtgaccgccgccgggatcactctcggcatggacgagctgtacaagtaa | 66 |

### Comparative example 1. Construction of vector comprising only epitope without carrier protein

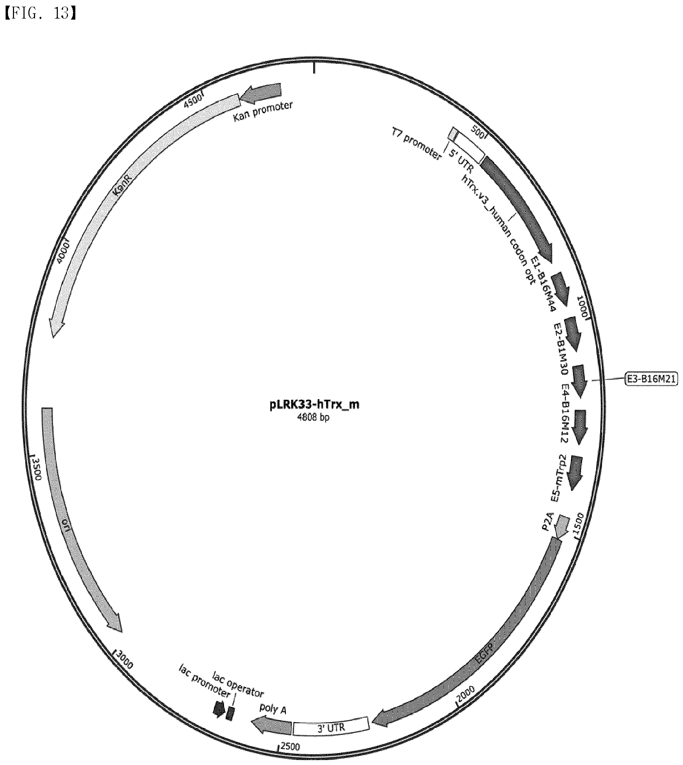

In order to prepare an insert capable of amplifying from the N-term of the epitope to the C-term of EGFP of Example 2 above, using NcoI_Epitope_F and XhoI_EGFP_R oligo of Table 16 below, by amplifying the corresponding part by performing PCR for the pLRK33-hTrx_m vector, the insert was produced. The vector map of the pLRK33-hTrx_m vector was shown in FIG. 13.

**[Table 16]**

| oligo type | Sequence (5' -> 3') | SEQ ID NO: |
|---|---|---|
| NcoI_Epitope_F | CCAAGGCCACCATGGAGTTCAAGCACATCAAGGC | 143 |
| XboI_EGFP_R | CTGTATTCTCGAGTTTACTTGTACAGCTCGT | 144 |

The produced insert was inserted into the NcoI, XhoI site of the pLRK33-empty vector of FIG. 10 using restriction enzymes NcoI (Thermo Scientific^{™} FastDigest; #FD0574) and XhoI (Scientific^{™} FastDigest; #FD0694) and T4 DNA ligase (Thermo Scientific^{™}; #EL0012), thereby producing a comparative example vector, pLRK33-No_CP_m, in which no carrier protein was inserted.

### Example 3. Construction of mRNA of carrier proteins comprising epitopes

mRNA was constructed by conducting in vitro transcription using 16 kinds of the vectors produced in Example 2 and Comparative example 1 above (pLRK33-hTrx_WT, pLRK33-CP12_m, pLRK33-CP13_m, pLRK33-CP23_m, pLRK33-CP31_m, pLRK33-CP33_m, pLRK33-CP34_m, pLRK33-CP36_m, pLRK33-CP38_m, pLRK33-CP47_m, pLRK33-CP48_m, pLRK33-CP50_m, pLRK33-CP136_m, pLRK33-CP201_m, pLRK33-CP202_m and pLRK33-No_CP_m) as templates.

Specifically, before conducting in vitro transcription, the vector was linearized by cutting the back part of poly A with XbaI (Termo Scientific, FD0684), so that only the desired region could be transcribed into RNA.

Using 15 kinds of the linearized vectors, under the conditions of Table 17 below, in vitro transcription was conducted, and DNase I (Invitrogen, AM2238) was treated to remove template DNA, and it was purified using RNeasy Mini kit (Qiagen, 74104), thereby completing construction of mRNA.

[Structural formula 2] 5'UTR - (Carrier protein 1) - L-E-L-E-L-E-L-E-L-E-L - (Carrier protein 2) - P2A - EGFP - 3'UTR - poly A

(In the structural formula 2, L refers to the linker sequence GGSGGGGSGG, and Carrier protein 1 (CP #1) and Carrier protein 2 (CP #2) correspond to each carrier protein inserted into the N-terminus and C-terminus of the produced vector, respectively)

**[Table 17]**

| Component | Volume or amount |
|---|---|
| 10X RNA polymerase buffer (NEB #M0460T) | 2 µl |
| CleanCap AG (3' Ome) (Trilink #N-7413) | 0.8 µl |
| 100 mM ATP (Jena Bioscience # RNT-107-L) | 1.8 µl |
| 100 mM CTP (Jena Bioscience # RNT-107-L) | 1.8 µl |
| 100 mM GTP (Jena Bioscience # RNT-107-L) | 1.8 µl |
| 100 mM N1-methyl pseudo UTP (Jena Bioscience # RNT-107-L) | 1.8 µl |
| 1M DTT (Jena Bioscience # RNT-107-L) | 0.8 µl |
| T7 RNA Polymerase High concentration (NEB #M0460T) | 0.5 µl |
| Murine RNase Inhibitor (NEB #M0314L) | 0.5 µl |
| DNA template | 1 µg |
| DNase/RNase-free water | Up to 20 µl |
| Total volume | 20 µl |

### Test example 1. Measurement of expression efficiency of carrier proteins for epitopes in K562 cell line

Some of the K562 cell line (ATCC, CCL-243) maintained in a tank was obtain, and after cell down, it was resuspended with complete media (RPMI1640, Gibco, 11875-093, 10% FBS, Gibco, 26140-079 and 1% Penicillin-Streptomycin, Gibco, 10378-016), and cell count was performed, and the complete media was further added or cell down was conducted again so that the concentration was to be 3*10⁵ cells/ml, and then it was resuspended with complete media, 20 ml of the media of which concentration was adjusted to 3*10⁵ cells/ml was cultured in a CO₂ incubator.

The above cultured K562 cell line was obtained, and electroporation with the mRNA prepared in Example 3 was performed into the cell line using the SF cell line 4D-Nucleofector X kit S (Lonza, V4XC-2032) according to the Amaxa 4D-Nucleofector protocol for K562, and cell healing was performed. The amount of mRNA used for transfection was 1 pmol per sample.

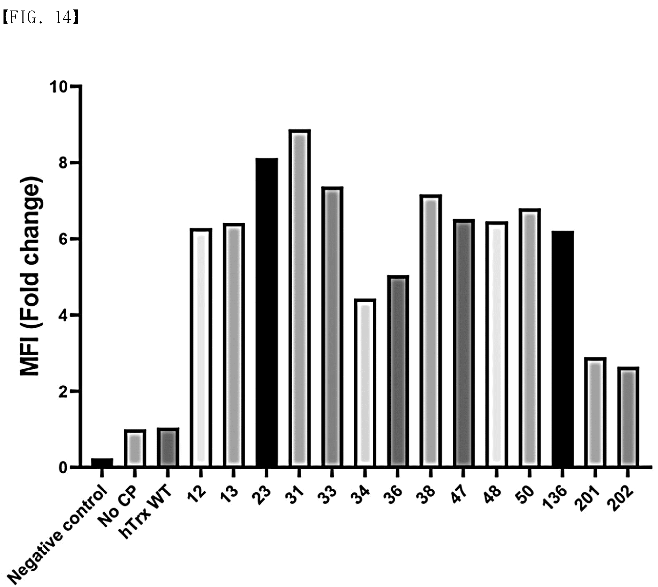

Cells were harvested in each well and cell down was performed, and then they were resuspended with FACS buffer (eBioscience, 00-4222-28) and moved to a FACS tube, and cell analysis was performed with LSRFortessa (BD Biosciences) to confirm the expression level of the epitope, and the result was shown in FIG. 14 and Table 18. As a negative control group, a cell line in which mRNA was not transfected was prepared, and the epitope expression level was confirmed.

**[Table 18]**

| | Mean fluorescence intensity | MFI (Fold change) |
|---|---|---|
| Negative control | 842 | 0.236849508 |
| No CP | 3555 | 1 |
| CP12 | 22325 | 6.279887482 |
| CP13 | 22827 | 6.421097046 |
| CP23 | 28865 | 8.11954993 |
| CP31 | 31535 | 8.870604782 |
| CP33 | 15108 | 4.24978903 |
| CP34 | 26188 | 7.36652602 |
| CP36 | 15746 | 4.429254571 |
| CP38 | 17939 | 5.046132208 |
| CP47 | 25447 | 7.158087201 |
| CP48 | 23171 | 6.517862166 |
| CP50 | 22971 | 6.461603376 |
| CP136 | 22103 | 6.217440225 |
| CP201 | 10259 | 2.885794655 |
| CP202 | 9405 | 2.64556962 |

As a result of the cell analysis, it was confirmed that the EGFP expression efficiency was increased overall in each CP combination comprising the carrier protein compared to No CP having no carrier protein, and it was confirmed that the expression of the epitope could be increased using the CP combination.

### Test example 2. Measurement of expression efficiency of carrier proteins for epitopes in human dendritic cells

Cryopreserved human PBMCs (Peripheral Blood Mononuclear Cells) (Stemexpress, PBMNC300C) stored in a liquid nitrogen tank were thawed according to the manufacturer' s protocol, and cell count was performed for the thawed cells, and based on the corresponding result, the cells were isolated according the user' s manual using human CD14 microbeads (Miltenyi Biotec, 130-050-201) and LS column (Miltenyi Biotec, 130-042-401) (using buffer by 2 ml for column activation, 2 ml for cell loading, 2 ml for wash 3 times, 5 ml for cell elution, respectively), and the number of the selected cells was measured.

The monocytes of the isolated cells were suspended with media enclosed in ImmunoCult dendritic cell culture kit (DC culture kit) (Stemcell, 10985) with media (w/ differentiation supplement) so that the concentration was to be 1e06 cells/ml, to produce monocyte derived dendritic cells (moDCs), and they were seeded by 100 µl per 1 well in a total of 8 wells in a 96 well plate.

The media of the cells which were being cultured were removed using the DC culture kit, and it was replaced by 100µl each with media in which the differentiation supplement enclosed in the DC culture kit was added, and the DC maturation supplement enclosed in the DC culture kit was added to the cell media by calculating the amount according to the user' s manual.

According to Table 19 below, the mRNA produced in Example 3 above, DP buffer and carrier were added in order and mixed. The carrier is a composition for drug delivery comprising the cationic compound and anionic polymer compound and the like of Example 1 of Laid-open patent publication no. 10-2024-0035317.

At each mixing step, pipetting was performed using a pipet to ensure sufficient mixing, and a drug product (DP) mixed with mRNA and a carrier was prepared by pipetting was performed 10 times immediately after adding the formulation. The prepared DP was treated to the cell media in which the DC maturation supplement was added and cultured depending on the amount of the treated DP of Table 19 below. Additionally, a negative control group in which no DP was treated was prepared.

**[Table 19]**

| | mRNA (1mg/ml stock) | DP Buffer | Carrier | DP |
|---|---|---|---|---|
| Negative control group | - | - | - | - |
| No CP | 10 µl | 30.4 µl | 9.6 µl | 1.46 µl |
| CP23 | | | | 1.72 µl |
| CP31 | | | | 1.78 µl |
| CP33 | | | | 1.67 µl |
| CP38 | | | | 1.68 µl |
| CP48 | | | | 1.84 µl |
| CP50 | | | | 1.92 µl |

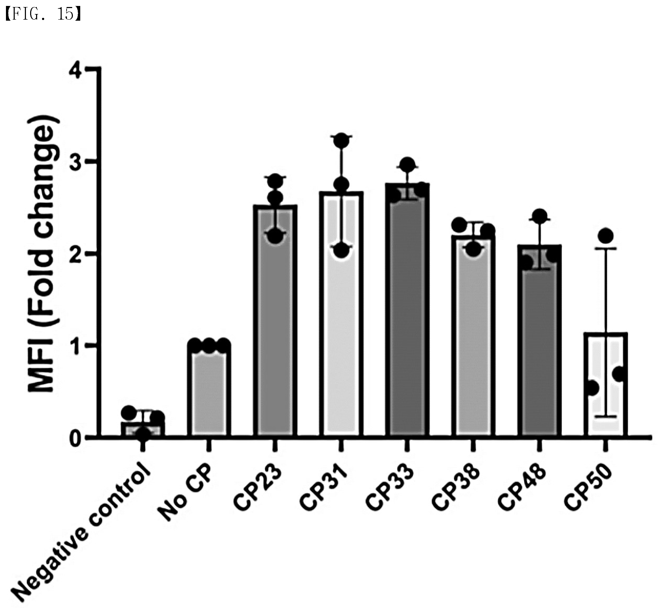

The DP was treated and the cells were harvested from each well and cell down was performed. After that, they were resuspended in a solution in which FACS buffer (eBioscience, 00-4222-28) and 7-AAD (BD biosciences, 559925) were mixed at a volume ratio of 100:1 and moved to a FACS tube, and cell analysis was performed with LSRFortessa (BD Biosciences) 3 times, and the result was shown in FIG. 15 and Table 20.

**[Table 20]**

| | MFI (Fold change) |
|---|---|
| Negative control | 0.173425 |
| No CP | 1 |
| CP23 | 2.528538 |
| CP31 | 2.673939 |
| CP33 | 2.763781 |
| CP38 | 2.204154 |
| CP48 | 2.098567 |
| CP50 | 1.144419 |

(The values of Table 20 above are mean values of MFI obtained by performing the cell analysis 3 times)

As a result of the cell analysis, MFI values of 2 folds or more were shown in the CP23, CP31, CP33, CP38 and CP48 combinations compared to no CP, and thus, it was confirmed that the human epitope expression was increased.

### Test example 3. Measurement of ability of inducing immunogenicity of carrier proteins for epitopes in human dendritic cells

The DP according to Table 19 above was produced by the substantially same method as Test example 2 above and was treated to media culturing the moDC, and was cultured for 2 days.

On the day when the culture was completed, the human PBMCs of Test example 2 used for moDC production were prepared separately, and the number of cells of the initial PBMCs was counted, and based on this, CD8+ T cells were isolated according to the user's manual using human CD8+ T cell isolation kit (Miltenyi Biotec, 130-096-495) and LS column (Miltenyi Biotec, 130-042-401).

The cultured moDC and CD8+ T cells in which isolation was completed were harvested and then cell down was conducted, and they were resuspended in Immunocult-XF T cell expansion medium (Stemcell, 10981), so as to be 5e05 cells/ml for the moDC and 2e06 cells/ml for the CD8+ T cells, respectively. After that, each cell suspension was mixed so as to be a volume ratio of 1:1, and 2 ml per well was seeded in a 6 well plate, and the moDC, CD8+ T cell mixture was cultured for 7 days.

The culture-completed cells were harvested again, and were resuspended so as to be 1e06 cell/ml using CTL media enclosed in mouse IFN-γ single-color ELISPOT kit (Immunospot, mIFNgp-2M).

Separately from the above process, each peptide corresponding to 5 kinds of the epitopes of Table 21 below was diluted to be CTL media 20 µM to produce restimulation peptide media, and the prepared cell suspension and peptide media were aliquoted by 100 µl each to each well in a 96 well plate for ELISPOT enclosed in the ELISPOT kit, and they were cultured for 16 hours or more. As a negative control group, they were cultured without adding peptide media.

**[Table 21]**

| Peptide type | Peptide sequence | SEQ ID NO: |
|---|---|---|
| NY-ESO-1 | SLLMWITQC | 147 |
| MAGE-A3 | FLWGPRALV | 148 |
| Survivin | LMLGEFLKL | 149 |
| Multi MAGE-A | YLEYRQVPV | 150 |
| Melan A | ELAGIGILTV | 151 |

| | | |
|---|---|---|
| (In Table 21 above, Multi MAGE-A refers to an antigen sequence commonly showing in various subfamily antigens of MAGE-A) | | |

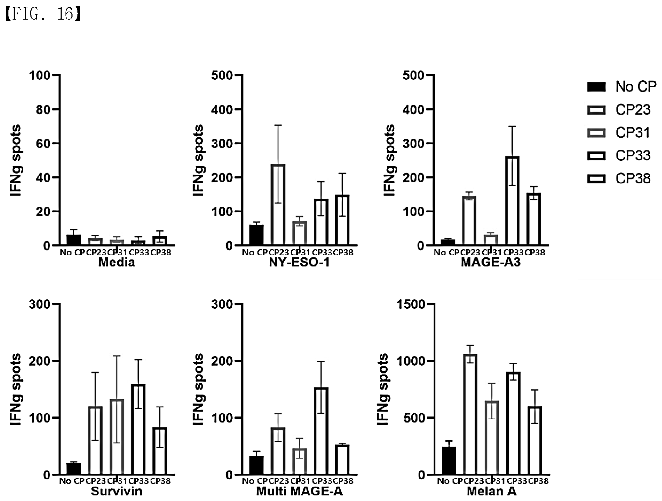

After culturing, a detection reaction was performed according to the user' s manual, and it was analyzed using ELISPOT plate analyzer (Immunospot), and the result was shown in FIG. 16.

As a result of the ELISPOT analysis, it was confirmed that the immunogenicity for multiple epitopes was increased in the CP23, CP31, CP33 and CP38 combinations compared to no CP.

## Claims

1. A conjugate, comprising mRNA of an antigen protein;
mRNA encoding a first carrier protein linked to the 5' end of the mRNA of the antigen protein; and
mRNA encoding a second carrier protein linked to the 3' end of the mRNA of the antigen protein.

2. The conjugate according to claim 1, wherein the first carrier protein and the second carrier protein are same or different, and are at least one selected from Ub (ubiquitin), UbR (ubiquitin A77R mutant), Lamp, mODC (mouse ornithine decarboxylase), cmyc, SORT1 (sortilin 1), TRX (Thioredoxin), PSBD (peripheral subunit-binding domain), TRXwt-UbR, TRXwt-Lamp, TRXwt-mODC, TRXwt-cmyc, TRXwt-SORT1, SORT1 w/TM and Lamp w/TM, respectively.

3. The conjugate according to claim 2, wherein the first carrier protein and the second carrier protein are selected from protein combinations shown in Table 1 as follows:
**[Table 1]**
| CP_set | N-term (First carrier protein) | C-term (Second carrier protein) |
|---|---|---|
| CP01 | TRXmt | PSBD |
| CP11 | UbR | mODC |
| CP12 | UbR | SORT1 |
| CP13 | Lamp | mODC |
| CP14 | Lamp | SORT1 |
| CP21 | UbR | cmyc |
| CP22 | UbR | Lamp |
| CP23 | mODC | SORT1 |
| CP24 | cmyc | mODC |
| CP25 | cmyc | SORT1 |
| CP26 | Lamp | Ub |
| CP27 | Lamp | cmyc |
| CP28 | SORT1 | mODC |
| CP31 | mODC | Ub |
| CP32 | mODC | cmyc |
| CP33 | mODC | Lamp |
| CP34 | cmyc | Ub |
| CP35 | cmyc | Lamp |
| CP36 | SORT1 | Ub |
| CP37 | SORT1 | cmyc |
| CP38 | SORT1 | Lamp |
| CP41 | TRXwt | cmyc |
| CP42 | TRXwt | Lamp |
| CP43 | TRXwt | mODC |
| CP44 | TRXwt | SORT1 |
| CP45 | TRXwt | Ub |
| CP46 | cmyc | TRXwt |
| CP47 | Lamp | TRXwt |
| CP48 | mODC | TRXwt |
| CP49 | SORT1 | TRXwt |
| CP50 | UbR | TRXwt |
| CP112 | TRXwt-UbR | SORT1 |
| CP113 | TRXwt-Lamp | mODC |
| CP123 | TRXwt -mODC | SORT1 |
| CP131 | TRXwt -mODC | Ub |
| CP133 | TRXwt -mODC | Lamp |
| CP134 | TRXwt-cmyc | Ub |
| CP136 | TRXwt-SORT1 | Ub |
| CP138 | TRXwt-SORT1 | Lamp |
| CP201 | TRXwt | SORT1 w/ TM |
| CP202 | Lamp w/ TM | TRXwt |

4. The conjugate according to claim 2,
wherein the UbR comprises the amino acid sequence of SEQ ID NO: 1;
the Ub comprises the amino acid sequence of SEQ ID NO: 2;
the Lamp comprises the amino acid sequence of SEQ ID NO: 3 or 4;
the mODC comprises the amino acid sequence of SEQ ID NO: 5 or 6;
the cmyc comprises the amino acid sequence of SEQ ID NO: 7 or 8;
the SORT1 comprises the amino acid sequence of SEQ ID NO: 9 or 10;
the TRXwt comprises the amino acid sequence of SEQ ID NO: 11 or 12;
the PSBD comprises the amino acid sequence of SEQ ID NO: 13;
the TRXwt-UbR comprises the amino acid sequence of SEQ ID NO: 14;
the TRXwt-Lamp comprises the amino acid sequence of SEQ ID NO: 15;
the TRXwt-mODC comprises the amino acid sequence of SEQ ID NO: 16;
the TRXwt-cmyc comprises the amino acid sequence of SEQ ID NO: 17;
the TRXwt-SORT1 comprises the amino acid sequence of SEQ ID NO: 18;
the SORT1 w/ TM comprises the amino acid sequence of SEQ ID NO: 19;
the Lamp w/ TM comprises the amino acid sequence of SEQ ID NO: 20; or
the TRXmt comprises the amino acid sequence of SEQ ID NO: 145.

5. The conjugate according to claim 3, wherein the first carrier protein and the second carrier protein are selected from protein combinations shown in Table 22 as follows:
**[Table 22]**
| CP_set | N-term (First carrier protein) | C-term (Second carrier protein) |
|---|---|---|
| CP12 | UbR | SORT1 |
| CP13 | Lamp | mODC |
| CP23 | mODC | SORT1 |
| CP31 | mODC | Ub |
| CP33 | mODC | Lamp |
| CP34 | cmyc | Ub |
| CP36 | SORT1 | Ub |
| CP38 | SORT1 | Lamp |
| CP47 | Lamp | TRXwt |
| CP48 | mODC | TRXwt |
| CP50 | UbR | TRXwt |
| CP136 | TRXwt-SORT1 | Ub |
| CP201 | TRXwt | SORT1 w/ TM |
| CP202 | Lamp w/ TM | TRXwt |

6. The conjugate according to claim 2, wherein the antigen protein comprises a T cell epitope.

7. The conjugate according to claim 6, wherein the T cell epitope is derived from a tumor antigen, an antigen of an infection source, an autoantigen, or an allergy causing antigen.

8. The conjugate according to claim 7, wherein the tumor antigen comprises a tumor-associated antigen (TAA), a tumor-specific antigen (TSA) or a tumor-derived neoantigen.

9. The conjugate according to claim 8, wherein the tumor-derived neoantigen comprises a mutation specifically expressed in a cancer cell.

10. The conjugate according to claim 8, wherein the tumor-associated antigen (TAA) is mTrp2, CT (Cancer-testis) antigen, EGFR, Ova, Melan-A, PSMA (Prostate Specific Membrane Antigen), Survivin, ADAbp (adenosine deaminase-binding protein), cyclophilin b, gp100, CRC (Colorectal associated antigen)-C017-1 A/GA733, CEA (carcinoembryonic antigen), CAP-1, CAP-2, etv6, AML1, PSA (Prostate Specific Antigen), PSA-1, PSA-2, PSA-3, MAGE (melanoma antigen E), GAGE (G antigen), BAGE (B melanoma antigen), RAGE (renal tumor antigen), LAGE (L antigen), NAG, GnT-V, MUM-1, CDK4, p53, tyrosinase, Muc1 (mucin1), HER2/neu, p21ras, N-RAS, K-RAS, RCAS1, α -fetoprotein, E-cadherin, α -catenin, β -catenin, γ-catenin, p120ctn, PRAME, NY-ESO-1, TRP2, Mammaglobin-A, metallopanstimulin-1 (MPS-1), cytochrome P450 isoform 1B1, 90K/Mac-2 binding protein, Ep-CAM (MK-1), HSP-70, hTERT (TRT), LEA, TAGE-1, 5T4, gp70, SCP-1, cmyc, cyclin B1, MDM2, p62, Koc, IMP1, TA90, OA1, CT-7, HOM-MEL-40/SSX-2, SSX-1, SSX-4, HOM-TES-14/SCP-1, HOM-TES-S5, HDAC5, MBD2, TRIP4, NY-CO-45, KNSL6, HIP1R, Seb4D, KIAA1416, IMP1, 90K/Mac-2 binding protein, MDM2, or LMNA.

11. The conjugate according to claim 8, wherein the tumor-specific antigen (TSA) is M12, M20, M21, M30 or M44.

12. The conjugate according to claim 7, wherein the antigen of the infection source is an antigen derived from a virus, bacterium, parasite, or fungus.

13. The conjugate according to any one claim of claim 1 to claim 12, wherein the conjugate has at least one characteristic selected from the group consisting of the following (i) and (ii) by the carrier protein:
(i) delivery of CD4/CD8 through a processing and presentation pathway, respectively; and
(ii) increase in expression of the antigen protein.

14. A composition for immunization, comprising the conjugate of any one claim of claim 1 to claim 12.

15. The composition for immunization according to claim 14, wherein the composition for immunization further comprises at least one selected from the group consisting of antigen adjuvants, efficacy enhancers, preservatives, buffers, surfactants, carriers, osmagents, anti-oxidants and stabilizers.

16. The composition for immunization according to claim 14, which is administered through at least one route selected from the group consisting of intranasal, intratracheal, oral, intradermal, intramuscular, intraperitoneal, intravenous, conjunctival and subcutaneous routes.

17. The composition for immunization according to claim 14, wherein the composition for immunization is a composition for immunization against cancer.

18. A method of immunization against cancer, comprising administering the conjugate according to any one claim of claim 1 to claim 12 or a vaccine composition comprising the same into a subject.
